(19) Europäisches Patentamt
European Patent Office
Office européen des brevets

(11) EP 3 056 902 B1

(12) EUROPEAN PATENT SPECIFICATION

(45) Date of publication and mention
of the grant of the patent:
**01.04.2020 Bulletin 2020/14**

(51) Int Cl.:
*G01N 33/542* (2006.01) *C07K 14/705* (2006.01)
*G01N 33/68* (2006.01)

(21) Application number: **15155202.3**

(22) Date of filing: **16.02.2015**

(54) **Novel voltage dependent ion channel fusions and method of use thereof**

Neuartige spannungsabhängige Ionenkanalfusionen und Verwendungsverfahren dafür

Nouvelles fusions de canal ionique dépendant de la tension et son procédé d'utilisation

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**17.08.2016 Bulletin 2016/33**

(73) Proprietors:
• **UNIVERSITE DE BORDEAUX**
**33000 Bordeaux (FR)**
• **CENTRE NATIONAL DE LA RECHERCHE SCIENTIFIQUE**
**75794 Paris Cedex 16 (FR)**
• **Institut Polytechnique de Bordeaux**
**33402 Talence Cedex (FR)**

(72) Inventors:
• **Ruigrok, Hermanus**
**33200 Bordeaux (FR)**
• **Percherancier, Yann**
**33140 Villenave d'Ornon (FR)**
• **Veyret, Bernard**
**33600 PESSAC (FR)**

(74) Representative: **Lecca, Patricia S.**
**Cabinet Lecca**
**21, rue de Fécamp**
**75012 Paris (FR)**

(56) References cited:
**WO-A1-2004/057333    WO-A1-2005/052186**
**US-B1- 6 660 844**

• R. FLYNN ET AL: "Targeting the Transient Receptor Potential Vanilloid Type 1 (TRPV1) Assembly Domain Attenuates Inflammation-induced Hypersensitivity", JOURNAL OF BIOLOGICAL CHEMISTRY, vol. 289, no. 24, 13 June 2014 (2014-06-13), pages 16675-16687, XP055196867, ISSN: 0021-9258, DOI: 10.1074/jbc.M114.558668

• RIEKO SAKAI ET AL: "Design and characterization of a DNA-encoded, voltage-sensitive fluorescent protein", EUROPEAN JOURNAL OF NEUROSCIENCE, vol. 13, no. 12, 1 June 2001 (2001-06-01), pages 2314-2318, XP055197762, ISSN: 0953-816X, DOI: 10.1046/j.0953-816x.2001.01617.x

• JENSEN A A ET AL: "Probing intermolecular protein-protein interactions in the calcium-sensing receptor homodimer using bioluminescence resonance energy transfer (BRET)", EUROPEAN JOURNAL OF BIOCHEMISTRY, WILEY-BLACKWELL PUBLISHING LTD, GB, vol. 269, no. 20, 1 October 2002 (2002-10-01), pages 5076-5087, XP002276727, ISSN: 0014-2956, DOI: 10.1046/J.1432-1033.2002.03218.X

• BACART J ET AL: "The BRET technology and its application to screening assays", BIOTECHNOLOGY JOURNAL, WILEY-VCH VERLAG, WEINHEIM, DE, vol. 3, no. 3, 1 March 2008 (2008-03-01), pages 311-324, XP008134377, ISSN: 1860-6768, DOI: 10.1002/BIOT.200700222 [retrieved on 2008-01-28]

- **F. H. YU: "Overview of Molecular Relationships in the Voltage-Gated Ion Channel Superfamily", PHARMACOLOGICAL REVIEWS, vol. 57, no. 4, 1 December 2005 (2005-12-01), pages 387-395, XP055197570, US ISSN: 0031-6997, DOI: 10.1124/pr.57.4.13**
- **V. De-La-Rosa ET AL: "Coarse Architecture of the Transient Receptor Potential Vanilloid 1 (TRPV1) Ion Channel Determined by Fluorescence Resonance Energy Transfer", Journal of Biological Chemistry, vol. 288, no. 41, 11 October 2013 (2013-10-11), pages 29506-29517, XP055266443, US ISSN: 0021-9258, DOI: 10.1074/jbc.M113.479618**
- **Bernd Nilius ET AL: "The transient receptor potential family of ion channels", Genome Biology, vol. 12, no. 218, 17 March 2011 (2011-03-17), pages 1-11, XP055482412,**

Remarks:
The file contains technical information submitted after the application was filed and not included in this specification

**Description**

**FIELD OF THE INVENTION**

The present invention relates to novel voltage-dependent ion channel fusion subunits as disclosed in the claims, and to a functional bioluminescence resonance energy transfer (BRET) assay for screening in real time and characterizing candidate molecules or physical parameters for their ability to activate or inhibit voltage-dependent ion channels. The present invention also relates to a cell-based or cell-free composition comprising at least one voltage-dependent ion channel fusion subunit wherein a bioluminescent donor molecule and/or acceptor molecule are bound to either C-terminal or N-terminal, of said channel subunit. The combination of the voltage-dependent ion channel fusion subunit bound to bioluminescent donor and/or fluorescent acceptor molecule enables bioluminescence resonance energy transfer (BRET) to be used to detect change of conformation of the voltage-dependent ion channel subunit, thereby indicating activation or inhibition of said channel.

**BACKGROUND OF THE INVENTION**

A large number of central processes in plant and animal cells are wholly or partially controlled via changes in the intracellular concentrations of certain ions, for example the proton concentration, and changes in the membrane potential and the ion gradients across the membrane. Virtually all animal cells are surrounded by a membrane composed of a lipid bilayer with proteins embedded in it. The membrane serves as both an insulator and a diffusion barrier to the movement of ions. Ion transporter/pump proteins actively push ions across the membrane to establish concentration gradients across the membrane, and ion channels allow ions to move across the membrane down those concentration gradients. Ion pumps and ion channels are electrically equivalent to a set of batteries and resistors inserted in the membrane, and therefore create a voltage difference between the two sides of the membrane. Ion channels participate in, and regulate, cellular processes as diverse as the generation and timing of action potentials, energy production, synaptic transmission, secretion of hormones and the contraction of muscles, etc.

Voltage-dependent ion channels are membrane proteins that conduct ions at high rates regulated by the voltage across the membrane. They play a fundamental role in the generation and propagation of the nerve impulse and in cell homeostasis. The voltage sensor is a region of the protein bearing charged amino acids that relocate upon changes in the membrane electric field. The movement of the sensor initiates a conformational change in the gate of the conducting pathway thus controlling the flow of ions. The first voltage-dependent ion channel that was isolated and purified was extracted from the eel electroplax where there is a large concentration of Na channels. Several years later, the sequence of the eel Na channel was deduced from its mRNA. The first K channel sequence was deduced from the *Shaker* mutant of *Drosophila melanogaster.* Voltage-dependent ion channel proteins are selective for particular ions. Such ions include, for example, potassium, sodium, and calcium.

Voltage-dependent ion channels are present in every cell and are involved in generation of electrical activity and information processing. Accordingly, defect in their function can result in various conditions, such as heart arrhythmias, epilepsy, hypertension, etc... Many drugs exert their specific effects via modulation of ion channels. Examples include antiepileptic compounds which block voltage-dependent sodium channels in the brain, antihypertensive drugs which block voltage-dependent calcium channels in smooth muscle cells, and some stimulators of insulin release which block ATP-regulated potassium channels in the pancreas. Recent developments now allow ion channels, to be significantly more promising targets for drug discovery. Novel ion channels have been identified and are being targeted for the treatment of cancer, immune disorders and other diseases, thereby expanding the existing ion channel drug disease set well beyond CNS and cardiovascular disorders.

The amino acid sequence of a voltage-dependent ion channel protein across species is highly conserved. The proteins that function as voltage-gated ion channels have three remarkable properties that enable cells to conduct an electric impulse: (1) opening in response to changes in the membrane potential (voltage gating); (2) subsequent channel closing and inactivation; and (3) like all ion channels, exquisite specificity for those ions that will permeate and those that will not. Voltage-gated $K^+$ channels, such as the Shaker protein from *Drosophila,* are assembled from four similar subunits, each of which has six membrane-spanning $\alpha$ helices and a nonhelical P segment that lines the ion pore. The S4 $\alpha$ helix in each subunit acts as a voltage sensor. Voltage-gated $Na^+$ and $Ca^{2+}$ channels are monomeric proteins containing four homologous domains each similar to a $K^+$-channel subunit. The ion specificity of channel proteins is due mainly to coordination of the selected ion with specific residues in the P segments, thus lowering the activation energy for passage of the selected ion compared with other ions. Voltage-sensing $\alpha$ helices have a positively charged lysine or arginine every third or fourth residue. Their movement outward across the membrane, in response to a membrane depolarization of sufficient magnitude, causes opening of the channel. Voltage-gated $K^+$, $Na^+$, and $Ca^{2+}$ channel proteins contain one or more cytosolic domains that move into the open channel thereby inactivating it. It is postulated that voltage-gated channel proteins and possibly all $K^+$ channels evolved from a common ancestral gene.

Among the voltage-dependent ion channels, the transient receptor- potential (TRP) ion channels, are garnering interest both as mediators of sensory signals and, more recently, as novel drug targets. There are six TRP subfamilies, the main ones being TRPM, TRPV and TRPC. The TRPM subfamily has been called the most novel of the TRP subfamilies, given the potential roles of TRPM channels in cell division, cell migration, and calcium signaling. For example, TRPM1 and TRPM8 show promise as oncology targets, while TRPM2 and TRPM4 are touted as novel targets for immune disorders. It is thought that most TRPs function as homotetramers. The formation of heteromultimeric channels between members of the same subfamily or different subfamilies has been described in several cases (such as between the TRPCs), and this could potentially create a wide variety of channels. A typical TRP protein contains six putative transmembrane segments (S1 to S6) with a pore-forming reentrant loop between S5 and S6. Intracellular amino and carboxyl termini are variable in length and consist of a variety of domains. The large intracellular domain can be seen as a 'nested box' structure: a 'wire frame' outer shell acts as a sensor for activators and modulators, and a globular inner chamber might modulate ion flow. Another feature in the amino termini of many TRPs is the presence of ankyrin repeats, 33-residue motifs consisting of pairs of antiparallel $\alpha$-helices connected by $\beta$-hairpin motifs. The number of repeats in the ankyrin repeat domain (ARD) can vary between different TRPs: 3 to 4 in TRPCs, 6 in TRPVs, 14 to 15 in TRPAs and about 29 in TRPNs. Functionally, ARD seems to be connected with tetramerization of the channel and interactions with ligands and protein partners.

Amongst the other candidates, voltage-gated sodium channel is a protein embedded in the plasma membrane. This type of protein is found in the nerve and muscle cells and is used in the rapid electrical signaling found in these cells. The principle subunit of the voltage-gated sodium channel is a polypeptide chain of more than 1800 amino acids. When the amino acid sequence of any protein embedded in a membrane is examined, typically one or more segments of the polypeptide chain are found to be comprised largely of amino acids with non-polar side chains. Each of these segments coils is what is called a transmembrane domain, with a length approximately the width of the membrane. Moreover, within a transmembrane domain the side chains necessarily face outward where they readily interact with the lipids of the membrane. By contrast, the peptide bonds, which are quite polar, face inward, separated from the lipid environment of the membrane. In the case of the voltage-gated sodium channel, there are 24 such transmembrane domains in the polypeptide chain. Sodium channels comprise of one pore-forming $\alpha$ subunit, which may be associated with either one or two $\beta$ subunits. $\alpha$-Subunits consist of four homologous domains (I-IV), each containing six transmembrane segments (S1-S6) and a pore-forming loop. The positively charged fourth transmembrane segment (S4) acts as a voltage sensor and is involved in channel gating. The crystal structure of the bacterial NavAb channel has revealed a number of novel structural features compared to earlier potassium channel structures including a short selectivity filter with ion selectivity determined by interactions with glutamate side chains. Interestingly, the pore region is penetrated by fatty acyl chains that extend into the central cavity which may allow the entry of small, hydrophobic pore-blocking drugs. Auxiliary $\beta1$, $\beta2$, $\beta3$ and $\beta4$ subunits consist of a large extracellular N-terminal domain, a single transmembrane segment and a shorter cytoplasmic domain.

Voltage-gated calcium ($Ca^{2+}$) channels are key transducers of membrane potential changes into intracellular $Ca^{2+}$ transients that initiate many physiological events. $Ca^{2+}$ channels purified from skeletal muscle transverse tubules are complexes of $\alpha1$, $\alpha2$, $\beta$, $\gamma$, and $\delta$ subunits. The $\alpha1$ subunit is a protein of about 2000 amino acid residues in length with an amino acid sequence and predicted transmembrane structure like the previously characterized, pore-forming $\alpha$ subunit of voltage-gated sodium channels. The amino acid sequence is organized in four repeated domains (I-IV), which each contains six transmembrane segments (S1-S6) and a membrane-associated loop between transmembrane segments S5 and S6. The intracellular $\beta$ subunit has predicted $\alpha$ helices but no transmembrane segments, whereas the $\gamma$ subunit is a glycoprotein with four transmembrane segments, the cloned $\alpha2$ subunit has many glycosylation sites and several hydrophobic sequences, whilst the $\delta$ subunit is encoded by the 3' end of the coding sequence of the same gene as the $\alpha2$ subunit, and the mature forms of these two subunits are produced by posttranslational proteolytic processing and disulfide linkage.

The sperm-specific CatSper channel controls the intracellular $Ca^{2+}$ concentration ($[Ca^{2+}]_i$) and, thereby, the swimming behaviour of sperm. These are putative 6TM, voltage-gated, calcium permeant channels that are presumed to assemble as a tetramer of $\alpha$-like subunits and mediate the current. CatSper subunits are structurally most closely related to individual domains of voltage-activated calcium channels ($Ca_v$). CatSper1, CatSper2 and CatSpers 3 and 4, in common with a recently identified putative 2TM auxiliary CatSper$\beta$ protein and two putative 1TM associated CatSper$\gamma$ and CatSper$\delta$ proteins, are restricted to the testis and localised to the principle piece of sperm tail.

Two pore channel is a small family of 2 members putatively forming cation-selective ion channels. They are predicted to contain two Kv-style six-transmembrane domains, suggesting they form a dimer in the membrane. These channels are closely related to CatSper channels and, more distantly, to TRP channels.

Cyclic nucleotide-regulated channels or cyclic nucleotide-gated channels (CNG) are responsible for signaling in the primary sensory cells of the vertebrate visual and olfactory systems. CNG channels are voltage-independent cation channels formed as tetramers. Each subunit has 6TM, with the pore-forming domain between TM5 and TM6. CNG channels were first found in rod photoreceptors, where light signals through rhodopsin and transducin to stimulate phosphodiesterase and reduce intracellular cGMP level. This results in a closure of CNG channels and a reduced 'dark current'. Similar channels were found in the cilia of olfactory neurons and the pineal gland. The cyclic nucleotides bind to a domain in the C terminus of the subunit protein. Within this category, hyperpolarisation-activated, cyclic nucleotide-gated (HCN) channels are cation channels that are activated by hyperpolarisation at voltages negative to ~-50 mV. The cyclic nucleotides cAMP and cGMP directly activate the channels and shift the activation curves of HCN channels to more positive voltages, thereby enhancing channel activity. HCN channels underlie pacemaker currents found in many excitable cells including cardiac cells and neurons. In native cells, these currents have a variety of names, such as $I_h$, $I_q$ and $I_f$. The four known HCN channels have six transmembrane domains and form tetramers. It is believed that the channels can form heteromers with each other, as has been shown for HCN1 and HCN4.

Potassium channels function to conduct potassium ions down their electrochemical gradient, doing so both rapidly (up to the diffusion rate of $K^+$ ions in bulk water) and selectively (excluding, most notably, sodium despite the sub-angstrom difference in ionic radius). Biologically, these channels act to set or reset the resting potential in many cells. In excitable cells, such as neurons, the delayed counterflow of potassium ions shapes the action potential. Potassium channels have a tetrameric structure in which four identical protein subunits associate to form a fourfold symmetric ($C_4$) complex arranged around a central ion conducting pore (i.e., a homotetramer). Alternatively four related but not identical protein subunits may associate to form heterotetrameric complexes with pseudo $C_4$ symmetry. All potassium channel subunits have a distinctive pore-loop structure that lines the top of the pore and is responsible for potassium selective permeability. Potassium ion channels remove the hydration shell from the ion when it enters the selectivity filter. The selectivity filter is formed by a five residue sequence, TVGYG, termed the signature sequence, within the P loop of each subunit. This signature sequence is highly conserved, with the exception that an isoleucine residue in eukaryotic potassium ion channels often is substituted with a valine residue in prokaryotic channels. This sequence in the P-loop adopts a unique structure, having their electro-negative carbonyl oxygen atoms aligned toward the centre of the filter pore and form a square anti-prism similar to a water-solvating shell around each potassium binding site. The hydrophobic region of the channel is used to neutralize the environment around the potassium ion so that it is not attracted to any charges. In turn, it speeds up the reaction. A central pore or a central cavity, 10 Å wide, is located near the center of the transmembrane channel, where the energy barrier is highest for the transversing ion due to the hydrophobity of the channel wall. The water-filled cavity and the polar C-terminus of the pore helices ease the energetic barrier for the ion. Repulsion by preceding multiple potassium ions is thought to aid the throughput of the ions. The presence of the cavity can be understood intuitively as one of the channel's mechanisms for overcoming the dielectric barrier, or repulsion by the low-dielectric membrane, by keeping the $K^+$ ion in a watery, high-dielectric environment.

In 2006, Professor Okumura discovered voltage-gated hydrogen ion channels (proton channels), a voltage dependent membrane protein consisting of two modules: the voltage sensor and phosphatase. These ion channel molecules form conduction pathways for hydrogen ions in the cell membrane; however, they are not always open, opening only when the voltage sensor detects electric signals. The structure showed a 'closed Wagasa (Japanese umbrella)' shape with a long helix running through the cell membrane to the cytoplasm and featured a wide inner-accessible vestibule. Voltage sensing amino acids on the protein were located below the phenylalanine-containing "gascket" region in the center of membrane, indicating that structure was in resting state.

It follows naturally that detailed structural elucidation of ion channels and the development of high-throughput screening techniques will accelerate the discovery of effective and selective new ion channel drugs. Traditional screening methods (fluorescent dyes, radiometric flux, and binding assays) are indirect measures of ion channel activity. Electrophysiological techniques combined with pharmacology provide the most detailed and direct way to study ion channel function; however, conventional electrophysiological techniques do not meet the throughput demands of large compound library screening. New technologies, including automated electrophysiology and planar patch-clamp techniques, have also been tried and tested. However these methods are not yet performing at the high throughput speeds desired.

A major limitation of the patch clamp technique is its low throughput. Typically, a single, highly trained operator can test fewer than ten compounds per day using the patch clamp technique. Furthermore the technique is not easily amenable to automation, and produces complex results that require extensive analysis.

Förster resonance energy transfer (FRET), or simply resonance energy transfer (RET), is the non-radiative transfer of energy from an excited state donor molecule to a ground state acceptor molecule. Energy transfer efficiency is dependent on the distance between the donor and acceptor, the extent of the spectral overlap and the relative orientation of the acceptor and donor dipoles. In most cases both the fluorescent donor and acceptor are engineered variants of green fluorescent protein (GFP) from *Aequoria victoria.* The most widely used FRET pair is cyan fluorescent protein (CFP) as the donor alongside yellow fluorescent protein (YFP) as the acceptor and this FRET system has previously been used to quantify direct ligand binding by a number of GPCRs.

In bioluminescence resonance energy transfer (BRET), the donor fluorophore of FRET is replaced with a luciferase and the acceptor can be any suitable fluorophore. The use of a luciferase avoids the need for illumination as the addition of a substrate initiates bioluminescent emission and hence resonance energy transfer. FRET with odorant receptors has only previously been demonstrated for Class A (a2-adrenergic and parathyroid hormone), and Class B (secretin) GPCRs. De La Rosa et al. (Journal of Biological Chemistry, Vol. 288, No. 41, 11 October 2013, pages 29506-29517) also described several FRET experiments with the voltage-dependent ion channel subunit TRPV1 carrying two fluorescent labels YFP and CFP at the N-terminus and C-terminus of the ion channel, but were not able to show any changes of FRET signal upon activation.

The main disadvantages of FRET, as opposed to BRET, are the consequences of the required excitation of the donor with an external light source. BRET assays show no photo bleaching or photoisomerization of the donor protein, no photodamage to cells, and no light scattering or autofluorescence from cells or microplates, which can be caused by incident excitation light. In addition one main advantage of BRET over FRET is the lack of emission arising from direct excitation of the acceptor. This reduction in background should permit detection of interacting proteins at much lower concentrations than it is possible for FRET. The present inventors have surprisingly found that bioluminescence resonance energy transfer (BRET) is particularly efficient to detect a target compound using voltage-dependent ion channel fusion subunits.

The present inventors have thus developed an advanced, non-destructive, cell-based or cell-free composition real time assay technology that is perfectly suited for voltage-dependent ion channel receptor research, with specific emphasis on drug screening and further mapping of signal transduction pathways. The bioluminescence resonance energy transfer (BRET) assay optimized by present inventors for voltage dependent ion channel receptor research, takes advantage of a naturally occurring phenomenon, namely, the Förster resonance energy transfer between a luminescent donor and a fluorescent acceptor. The transfer efficiency depends on the degree of the spectral overlap, the relative orientation, and the distance between the donor and acceptor.

Since the fluorescent energy transfer of the invention is based on stimulatory principles such as BRET, a biosensor as described herein based on FRET instead of BRET would also be expected to function well and is included within the scope of the present invention.

## SUMMARY OF THE INVENTION

The present invention relates to novel nucleic acids encoding voltage-dependent ion channel fusion subunit comprising one or more bioluminescent donor molecule and/or one or more fluorescent acceptor molecules as disclosed in the claims. According to the present invention, voltage-dependent ion channel subunit may be voltage-dependent cation channel or voltage-dependent anion channel.

The present invention also relates to expression vectors comprising nucleotide sequences encoding the voltage-dependent channel fusion subunits, recombinant cells comprising such expression vectors, process for the production of voltage-dependent ion channel subunit, as well as to the voltage-dependent fusion subunits *per se.*

This invention further relates to a method of identifying a compound or a candidate capable of binding to a target domain of a voltage-dependent ion channel by providing a nucleic acid comprising a nucleotide sequence encoding a voltage-dependent ion channel fusion subunit bound to a nucleotide sequence encoding at least one bioluminescent donor molecule and/or bound to a nucleotide sequence encoding at least one fluorescent acceptor molecule. Given the complex structure of these voltage-dependent ion channels which can comprise from 2 to 24 transmembrane domains, it was surprising that such a fusion subunit with BRET donor and acceptor tags would retain structural and functional integrity. In a preferred embodiment of the present invention, the voltage-dependent ion channel subunits may comprise subunit of a voltage-dependent anion channel. In a further preferred embodiment, the voltage-dependent ion channel subunits may comprise subunit of a voltage-dependent cation channel. These channels can be of any source as long as when expressed in a cell, the N-terminus and C-terminus are intracytoplasmic. Examples include, but are not limited to, mammalian receptors, or biologically active variants or fragments thereof.

## BRIEF DESCRIPTION OF THE FIGURES

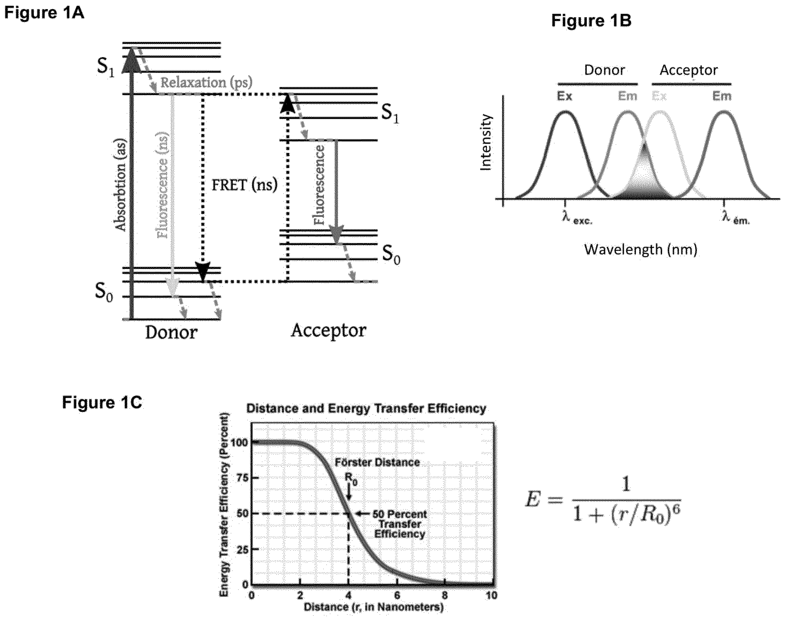

**Figures 1: (A)** is a schematic representation of a non-radiative transfer of energy between a donor and an acceptor. **(B)** is a schematic showing the overlapping of the wave lengths between donor and acceptor molecules. (C) is a schematic of the efficacy of the transfer function of the distance (r) between donor and acceptor molecules. (C) also presents the equation representing the efficacy of the transfer of energy E, wherein $R_0$ designates the distance of Förster for a specific donor-acceptor couple and is defined as being the distance allowing 50% of the maximal efficacy of transfer.

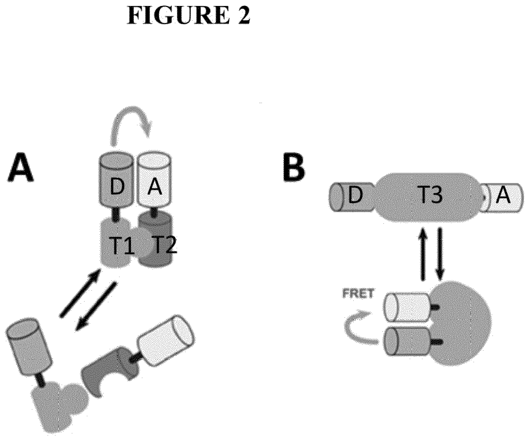

**Figures 2:** is a schematic representation of the intermolecular fluorescent probes (A) and intramolecular fluorescent probes (B). The donor molecule is represented as "D", the acceptor is represented as "A" and the molecules of interest are "T1", "T2", or "T3".

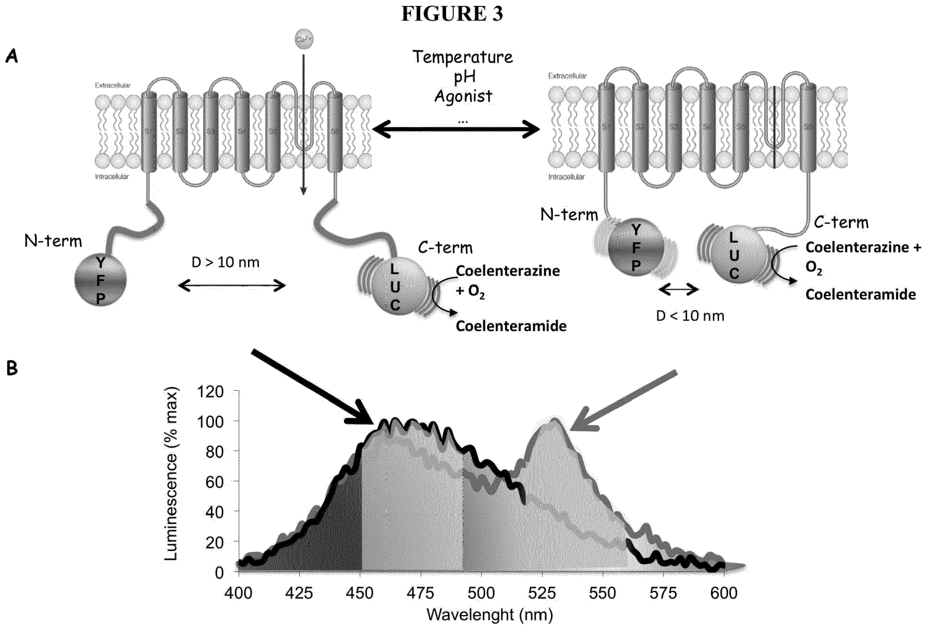

**Figures 3:** represents an example of use of an intramolecular BRET probe based on a voltage-dependent ion channel subunit according to the present invention. (A) Depending on the state of activation of the canal, the distance and/or the orientation between the N- and C- terminal ends of the voltage-dependent ion channel subunit were modified. (B) is a diagram of the spectra obtained in absence and in presence of BRET signal; Grey shadings within the graphs are representative of the light intensity of the acceptor on the right and the donor on the left.

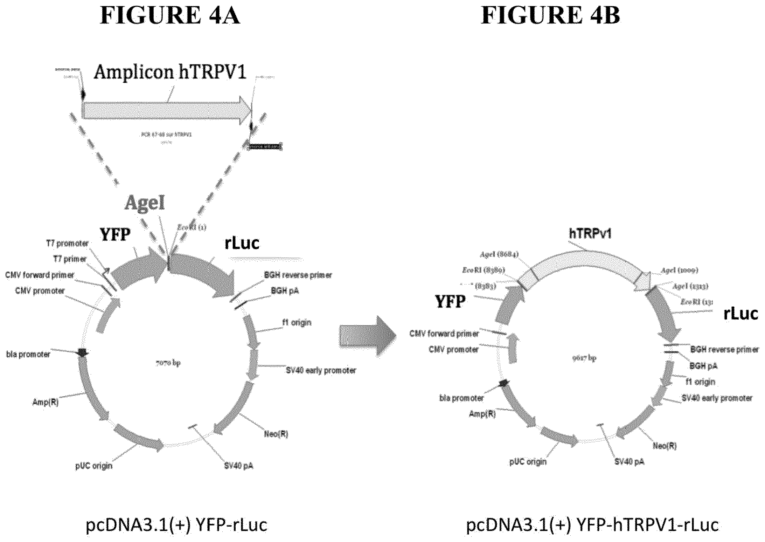

**Figures 4:** represents the cloning strategy used to obtain expression plasmid of fusion channel subunit YFP-hTRPV1-rLuc. After amplification of the cDNA encoding hTRPV1 with appropriate primers, the amplicon is inserted in fusion in-between YFP and rLuc in the restriction site Age I of an existing expression vector where YFP and rLuc are already cloned in fusion (A). The resulting expression vector is represented in the Figure (B). The same strategy was used to clone hTRPV3 fusion subunit with YFP and rLuc, except that the insertion was made in the restriction site EcoRI.

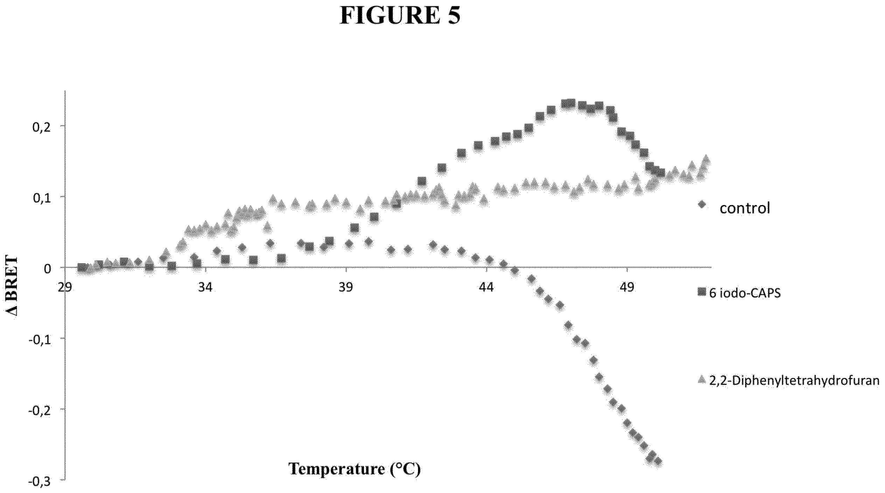

**Figure 5:** shows the evolution of the BRET signal over temperature in HEK293T cells transfected with YFP-hTRPV1-rLuc in control conditions (diamonds, n=10), in presence of 6 iodo-CAPS (squares, n=3) or with 2,2-Diphenyltetrahydrofuran (triangles, n=1). $\Delta_{BRET}$ represented the variation of BRET ratio over initial measure at 29.5 °C.

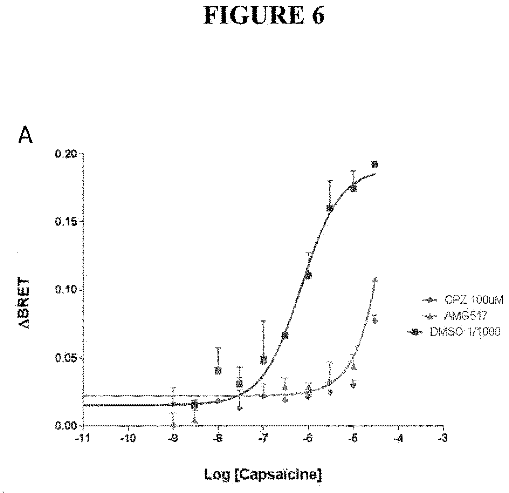

**Figure 6:** shows the dose-response of capsaïcin effect at 37 °C on BRET signal in HEK293T cells transfected with the probe YFP-hTRPV1-rLuc (n=3). 5 minutes before readings, cells were preincubated in presence of DMSO (as a control, square symbols), or either AMG517 (triangle symbols) or Capsazepine (CPZ) (diamond symbols), two well-known inhibitors of TRPV1.

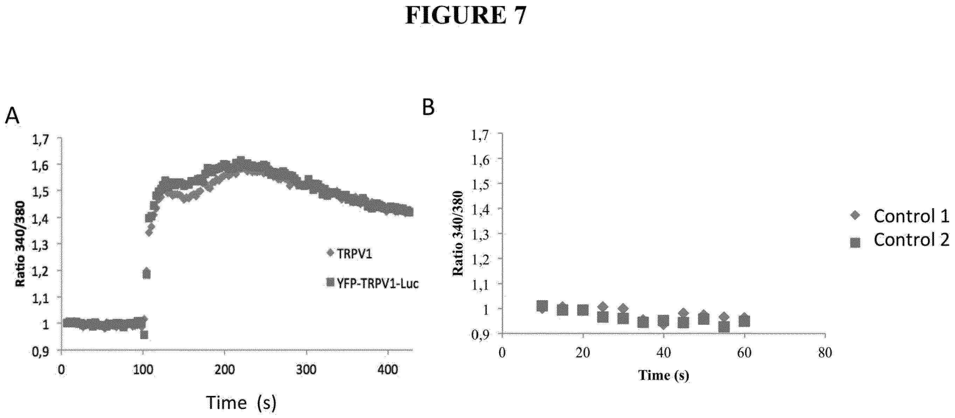

**Figure 7:** shows the measure of $Ca^{2+}$ at 37 °C in presence of 1 μM of capsaïcin injected after 100 sec (A) or 20 sec (B) in HEK293T cells transfected with hTRPV1 or with YFP-hTRPV1-rLuc (A) or with empty vector (B) and loaded with Fura 2.

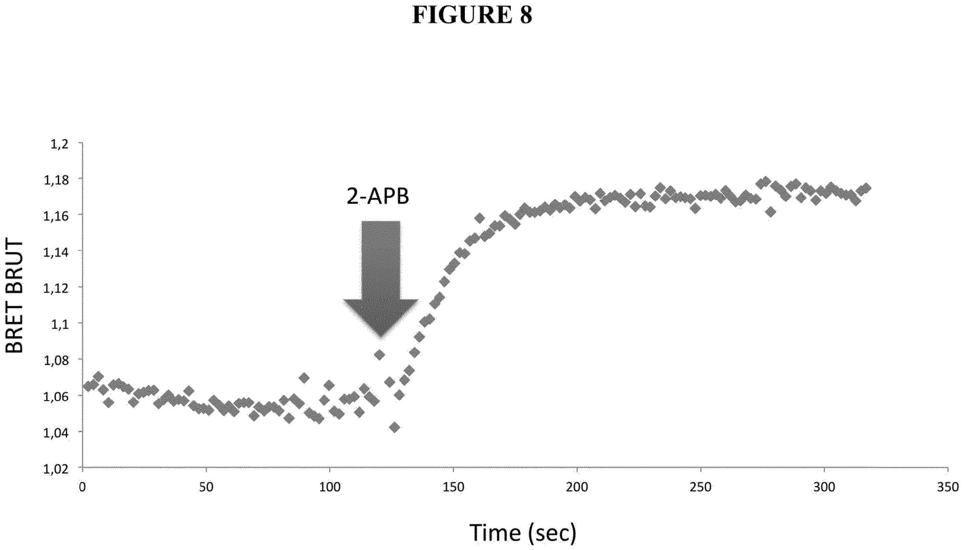

**Figure 8:** shows the kinetic of evolution of the BRET signal in HEK293T cells transfected with YFP-hTRPV3-rLuc before and after injection of 2-aminoethyl diphenylborinate in the culture media.

## DETAILED DESCRIPTION OF THE INVENTION

Unless specifically defined otherwise, all technical and scientific terms used herein shall be taken to have the same meaning as commonly understood by one of ordinary skill in the art (e.g., in cell culture, donor, acceptor, voltage-dependent ion channel, fusion subunits).

Unless otherwise indicated, the recombinant protein, cell culture, and molecular cloning techniques utilized in the present invention are standard procedures, well known to those skilled in the art. Such techniques are described and explained throughout the literature in sources such as, J. Perbal, A Practical Guide to Molecular Cloning, John Wiley and Sons (1984), J. Sambrook et al., Molecular Cloning: A Laboratory Manual, Cold Spring Harbour Laboratory Press (1989), T.A. Brown (editor), Essential Molecular Biology: A Practical Approach, Volumes 1 and 2, IRL Press (1991), D.M. Glover and B.D. Hames (editors), DNA Cloning: A Practical Approach, Volumes 1-4, IRL Press (1995 and 1996), and F. M. Ausubel et al. (editors), Current Protocols in Molecular Biology, Greene Pub. Associates and Wiley-Interscience (1988, including all updates until present), Ed Harlow and David Lane (editors) Antibodies: A Laboratory Manual, Cold Spring Harbour Laboratory, (1988), and J. E. Coligan et al. (editors) Current Protocols in Immunology, John Wiley & Sons (including all updates until present).

As used herein, the terms bioluminescent or fluorescent donor molecule refer to any molecule able to generate luminescence following either action on a suitable substrate, or its own excitation by an external source.

As used herein, the term substrate refers to any molecule that can be used in conjunction with a bioluminescent molecule to generate or absorb luminescence.

As used herein, the phrase allowing the bioluminescent or fluorescent donor molecule to modify the substrate refer to any enzymatic activity of the bioluminescent protein on the substrate that produces energy.

As used herein, the term acceptor molecule refers to any compound which can accept energy emitted as a result of the activity of a bioluminescent or fluorescent donor molecule, and re-emit it as light energy.

As used herein, bioluminescent resonance energy transfer (BRET) is a proximity assay based on the non-radiative transfer of energy between the bioluminescent donor molecule and the fluorescent acceptor molecule. As used herein, fluorescent resonance energy transfer (FRET) is a proximity assay based on the non-radiative transfer of energy between the fluorescent donor molecule and the fluorescent acceptor molecule.

As used herein, the term "modulate or modulation" or variations thereof refer to an alteration in the intensity and/or emission spectra of the bioluminescent donor and/or acceptor molecule.

As used herein, the term "spatial location" refers to the three dimensional positioning of the bioluminescent donor molecule relative to the acceptor molecule which changes as a result of the compound binding to the voltage-dependent ion channel or of the change in specific physical parameters.

As used herein, the term "dipole orientation" refers to the direction in three-dimensional space of the dipole moment associated either with the bioluminescent donor molecule and/or the acceptor molecule relative to their orientation in three-dimensional space. The dipole moment is a consequence of a variation in electrical charge over a molecule.

As used herein, the term "more sensitive" refers to a greater change in resonance energy transfer ratio between the ligand unbound form to the ligand bound form of one reporter system (for example, BRET) to another reporter system (for example, FRET).

As used herein, the term "contacting" refers to the addition of a candidate molecule and/or a sample capable of activating or inhibiting the voltage-dependent ion channel. The sample can be any substance or composition suspected of comprising a compound to be detected.

Voltage-dependent ion channels are a proven target for drug discovery, and many ion channel modulators are currently in clinical use for the treatment of pain, epilepsy, hypertension and other disease states. They comprise the molecular basis for essential physiological functions including fluid secretion, electrolyte balance, and bioenergetics and membrane excitability. Ion channels make good drug targets because they are physiologically essential, are pharmacologically accessible, are encoded by a variety of genes and usually operate as multimeric protein assemblies, resulting in a high degree of functional and anatomical specificity. Through molecular cloning, heterologous expression and electrophysiological characterization by patch-clamping, it is clear that the complexity of ion-channel biology also offers multiple opportunities for small-molecule drugs to achieve a specific, desired functional effect. For example, small molecules might influence a variety of biophysical properties of ion channels, such as voltage-dependence permeability, use-dependence, activation and inactivation. In contrast to simple blockers or openers, the discovery of modulatory compounds could allow the development of drugs that specifically act on cells or tissues exhibiting aberrant levels of ion-channel activity.

Modulators are any agents capable of altering the functional activity of voltage-dependent ion channel within a cell, and may represent a channel opener (functional agonist), a channel blocker (functional antagonist) or an agent that alters the level of expression of the channel at the cell membrane. An increase in expression will lead to greater numbers of voltage-dependent ion channels, which will lead to an increase in overall activity. Conversely, a decrease in expression will lead to a smaller overall activity. The major challenge in development of voltage-dependent ion channel modulators is to achieve selectivity for a specific target voltage-dependent ion channel over other related voltage-dependent ion channel subtypes, and for channels in the target tissue.

Voltage-dependent ion channels are known to exist in a number of different conformational states, referred to as gating states. For voltage-gated ion channels a channel can be viewed as residing in one of 3 gating states-closed (no ion permeation), open (ion flux occurs) and inactivated (no ion permeation; channel cannot be opened by depolarization), although it should be noted that some channels do not exhibit an inactivated state. Transition between gating states is voltage-dependent, and at any given time, equilibrium exists between these gating states, with the proportion of channels residing in each state depending upon the cellular membrane potential.

Many voltage-dependent ion channel modulators have been shown to bind preferentially to a specific gating state or states. For example, the voltage-gated sodium channel blocker Lamotrigine is thought to bind to the opened and inactivated states of the brain sodium channel protein. Preferential binding to a particular gating state may occur through an increase in channel affinity for the ion channel modulator, or simply through improved access of the drug to its binding site on the channel.

As used herein the terms "voltage-dependent ion channel fusion subunit" comprise a voltage-dependent ion channel subunit coupled or associated with at least a bioluminescent donor molecule and at least a fluorescent acceptor molecule. The bioluminescent donor molecule and the fluorescent acceptor molecule are preferably covalently attached, more preferably produced as a fusion protein with a subunit of the voltage-dependent ion channel or may be optionally coupled or fused to the subunit via a linker sequence. The bioluminescent donor molecule and the fluorescent acceptor molecule may be associated, either covalently attached or produced as a fusion protein to the same subunit or to different subunits of the same voltage-dependent ion channel. Alternatively, the bioluminescent donor molecule and the fluorescent acceptor molecule may be associated, either covalently attached or produced as a fusion protein of subunits of same or different subfamily of voltage-dependent ion channel. In addition, multiple combinations of bioluminescent donor molecule and the fluorescent acceptor molecule may be used to increase the sensitivity of the detection of tested compound candidates.

The bioluminescent donor molecule and the fluorescent acceptor molecule may completely replace or be inserted inside a specified region of the voltage-dependent ion channel. As the skilled person in the art will appreciate, the bioluminescent donor molecule and the fluorescent acceptor molecule are not inserted such that it makes the voltage-dependent ion channel inactive as result in a spatial change to the location and/or dipole orientation of the bioluminescent donor molecule relative to the fluorescent acceptor molecule.

By "substantially purified" or "purified" we mean a voltage-dependent channel subunit that has been separated from one or more lipids, nucleic acids, other polypeptides, or other contaminating molecules with which it is associated in its native state. It is preferred that the substantially purified polypeptide is at least 60% free, more preferably at least 75% free, and more preferably at least 90% free from other components with which it is naturally associated. However, at present there is no evidence that the polypeptides of the invention exist in nature.

The term "recombinant" in the context of a polypeptide refers to the polypeptide when produced by a cell, or in a cell-free expression system, in an altered amount or at an altered rate compared to its native state. In one embodiment, the cell is a cell that does not naturally produce the polypeptide. However, the cell may be a cell which comprises a non-endogenous gene that causes an altered, preferably increased, amount of the polypeptide to be produced. A recombinant polypeptide of the invention includes polypeptides which have not been separated from other components of the transgenic (recombinant) cell, or cell-free expression system, in which it is produced, and polypeptides produced in such cells or cell-free systems which are subsequently purified away from at least some other components.

The terms "polypeptide" and "protein" are generally used interchangeably and refer to a single polypeptide chain which may or may not be modified by addition of non-amino acid groups. It would be understood that such polypeptide chains may associate with other polypeptides or proteins or other molecules such as co-factors. The terms "proteins" and "polypeptides" as used herein also include variants, mutants, biologically active fragments, modifications, analogous and/or derivatives of the polypeptides described herein.

The % identity of a polypeptide is determined by GAP analysis (GCG program) with a gap creation penalty of 5, and a gap extension penalty of 0.3. The query sequence is at least 25 amino acids in length, and the GAP analysis aligns the two sequences over a region of at least 25 amino acids. More preferably, the query sequence is at least 50 amino acids in length, and the GAP analysis aligns the two sequences over a region of at least 50 amino acids. More preferably, the query sequence is at least 100 amino acids in length and the GAP analysis aligns the two sequences over a region of at least 100 amino acids. Even more preferably, the query sequence is at least 250 amino acids in length and the GAP analysis aligns the two sequences over a region of at least 250 amino acids. Even more preferably, the GAP analysis aligns the two sequences over their entire length.

As used herein, a "biologically active fragment" is a portion of a polypeptide as described herein, which maintains a defined activity of the full-length polypeptide. For example, a biologically active fragment of a voltage-dependent ion subunit must be capable of binding the target compound, resulting in a conformational change. Biologically active fragments can be any size as long as they maintain the defined activity. Preferably, biologically active fragments are at least 150, more preferably at least 250 amino acids in length.

As used herein, a "biologically active variant" is a molecule which differs from a naturally occurring and/or defined molecule by one or more amino acids but maintains a defined activity, such as defined above for biologically active fragments. Biologically active variants are typically least 50%, more preferably at least 80%, more preferably at least 90%, more preferably at least 95%, more preferably at least 97%, and even more preferably at least 99% identical to the naturally occurring and/or defined molecule.

With regard to a defined polypeptide or polynucleotide, it will be appreciated that % identity figures higher than those provided above will encompass preferred embodiments. Thus, where applicable, in light of the minimum % identity figures, it is preferred that the polypeptide or polynucleotide comprises an amino acid sequence which is at least 50%, more preferably at least 60%, more preferably at least 70%, more preferably at least 80%, more preferably at least 85%, more preferably at least 90%, more preferably at least 91%, more preferably at least 92%, more preferably at least 93%, more preferably at least 94%, more preferably at least 95%, more preferably at least 96%, more preferably at least 97%, more preferably at least 98%, more preferably at least 99%, more preferably at least 99.1%, more preferably at least 99.2%, more preferably at least 99.3%, more preferably at least 99.4%, more preferably at least 99.5%, more preferably at least 99.6%, more preferably at least 99.7%, more preferably at least 99.8%, and even more preferably at least 99.9% identical to the relevant nominated SEQ ID NO.

**[0050]** By an "isolated polynucleotide", including DNA, RNA, or a combination of these, single or double stranded, in the sense or antisense orientation or a combination of both, we mean a polynucleotide which is at least partially separated from the polynucleotide sequences with which it is associated or linked in its native state. Preferably, the isolated polynucleotide is at least 60% free, preferably at least 75% free, and most preferably at least 90% free from other components with which they are naturally associated.

In recent years, bioluminescence resonance energy transfer approaches (BRET) have been increasingly used to study protein-protein interactions and drug discovery. Luminescence in general is a phenomenon in which energy is specifically channeled to a molecule to produce an excited state. Return to a lower energy state is accompanied by release of a photon. Luminescence includes fluorescence, phosphorescence, chemiluminescence and bioluminescence. Bioluminescence is the process by which living organisms emit light that is visible to other organisms. Where the luminescence is bioluminescence, creation of the excited state derives from an enzyme catalyzed reaction. Luminescence can be used in the analysis of biological interactions. The dependence of the energy transfer efficacy on the distance between energy donors and acceptors permits real time measurements that are both sensitive and specific to the labeling sites of the proteins thus allowing inference on the dynamic structural changes.

One technique for assessing protein-protein interaction is based on fluorescence resonance energy transfer (FRET). The theory of FRET has been described for the first time in 1948 by Théodore Förster. It is defined as a non-radiatif transfer of energy, i.e., a transfer of energy which occurs without emission of photons and which results in a dipole interaction between a donor of energy and an acceptor of energy (Figure 1). Therefore, in this process, one fluorophore donor transfers its excited-state energy to another fluorophore acceptor. The non-radiative transfer of energy from the donor to the acceptor results in the excitation of the acceptor. The acceptor returns at basal stage by emitting a photon at its own length wave (Figure 1A).

According to Förster equation, FRET efficiency depends on five parameters: (i) the overlap - at least a partial overlap - between the absorption spectrum of the second fluorophore and the emission spectrum of the first fluorophore (Figures 1A and IB), (ii) the relative orientation between the emission dipole of the donor and the absorption dipole of the acceptor, (iii) the distance between the fluorophores (Figure IB), (iv) the quantum yield of the donor and the acceptor, and (v) the extinction coefficient of the acceptor.

These donor and acceptor fluorophores may thus be used as molecular probes, either intermolecular probes or intramolecular probes (Figure 2).

FRET has been used to assay protein-protein proximity in vitro and in vivo by chemically attaching fluorophores such as fluorescein and rhodamine to pairs of purified proteins and measuring fluorescence spectra of protein mixtures or cells that were microinjected with the labeled proteins. FRET, however, has several limitations. As with any fluorescence technique, photobleaching of the fluorophore and autofluorescence of the cells/tissue can significantly restrict the usefulness of FRET, and, in highly autofluorescent tissues, FRET is essentially unusable. Also, if the excitation light easily damages the tissue, the technique may be unable to give a value for healthy cells. Finally, if the cells/tissues to be tested are photoresponsive (*e.g.*, retina), FRET may be impractical because as soon as a measurement is taken, the photoresponse may be triggered.

In order to overcome the above disadvantages and further taking advantage of multiple sites of energy donor and acceptor insertions in the protein-protein complex of interest, the present invention relies on the development of a BRET-based assay that directly monitors real-time interactions between voltage dependent ion channels and potential agonists and antagonists.

The BRET method is very similar to that of FRET wherein the donor is a bioluminescent molecule. No external source of excitation is thus required, thereby avoiding inconveniences of photobleaching and autofluorescence of the cells/tissues. The BRET is actually a natural phenomenon which is produced in living organisms such as *Renilla reniformis and Aequorea victoria.* In those organisms, the luciferase enzyme, which is a bioluminescent protein, performs an oxidative degradation of the substrat cœlenterazin and at the same time emits some light with a peak at 480nm. In these organisms, the proximity to the Green Fluorescent Protein (GFP) allows a non-radiative transfer of energy. A good acceptor of energy may be the Yellow Fluorescent Protein (YFP) which is excited at 512nm and emits at 530nm. The BRET signal is measured by the ratio between the light intensity at the peak of emission of the acceptor and the light intensity at the peak of emission of the donor (Figure 3). The BRET signal must be subtracted to the signal obtained in presence of luciferase alone in the same experimental conditions in order to obtain the net BRET signal.

The present invention recognizes for the first time a BRET analysis of voltage regulated ion channels. The present invention includes instrumentation and methods that provide for the accurate and reliable information generation. An object of the present invention is to provide a screening system that targets ion channels and has superior efficiency. The present invention provides a material for screening for compounds that act on a target ion channel, comprising cells which retain at least one nucleic acid encoding a voltage-dependent channel.

The invention also provides a useful tool to probe for conformational changes occurring in the voltage dependent ion channel complexes resulting from ligand binding. As a result, by multiplexing different BRET-biosensors of the voltage dependent ion channel subunits, the invention offers the possibility to set up pharmacological fingerprints that are specific to each receptor ligand, thus allowing differentiating the distinct signaling modes of different ligands toward the various signaling pathways engaged.

High-throughput screening for ion-channel function requires sensitive, simple assays and instrumentation that will report ion channel activity in living cells.

The present invention thus relates to a nucleic acid or polynucleotide comprising a nucleotide sequence encoding a voltage-dependent ion channel fusion subunit bound to a nucleotide sequence encoding at least one bioluminescent or fluorescent donor molecule and/or bound to a nucleotide sequence encoding at least one fluorescent acceptor molecule.

The voltage-dependent ion channel subunit is a subunit of a voltage-dependent cation channel or voltage-dependent anion channel and comprises 2 to 24 transmembrane domains. Mammalian voltage dependent sodium channels have been identified and are well known in the art. According to the present invention, the voltage-dependent ion channels are described in the following Tables 1-11. These include also isoforms or active variants thereof.

According to the present invention, voltage-dependent ion channel subunit is a subunit of a voltage-dependent cation channel or voltage-dependent anion channel and comprises 2 to 24 transmembrane domains.

Table 1: Sodium channels, voltage-gated

| Approved Symbol | Approved Name | Tissue distribution | Synonyms | Chromosome | Accession number |
|---|---|---|---|---|---|
| SCN1A | sodium channel, voltage-gated, type I, alpha subunit | CNS | Nav1.1, GEFSP2, HBSCI, NAC1, SMEI | 2q24.3 | P35498, E9PG49 Q9C008, X03638 X65362, AF003372 |
| SCN1B | sodium channel, voltage-gated, type I, beta subunit | CNS- brain, skeletal muscle, and heart | | 19q13.12 | Q07699, Q5TZZ4, Q6TN97 M91808, L10338 |
| SCN2A | sodium channel, voltage-gated, type II, alpha subunit | CNS | Nav1.2, HBSCII, HBSCI | 2q24.3 | Q99250, A6NC14 A6NC14, X03639,X65361 M94055, X61149 |
| SCN2B | sodium channel, voltage-gated, type II, beta subunit | CNS | | 11q23.3 | 060939, 075302, Q9UNN3 U37026, U37147, AF007783 |
| SCN3A | sodium channel, voltage-gated, type III, alpha subunit | CNS | Nav1.3 | 2q24 | Q9NY46 , Q16142 Q9Y6P4 ,Y00766 |
| SCN3B | sodium channel, voltage-gated, type III, beta subunit | Heart | HSA243396 | 11q24.1 | Q06W27,A5H1I5, Q17RL3, Q9ULR2 |
| SCN4A | sodium channel, voltage-gated, type IV, alpha subunit | skeletal muscle-denervated and innervated skeletal muscle | Nav1.4, HYPP, SkM1 | 17q23.3 | P35499, Q15478, Q16447, Q7Z6B1 M26643, M81758 |
| SCN5A | sodium channel, voltage-gated, type V, alpha subunit | denervated skeletal muscle, heart | Nav1.5, LQT3, HB1, HBBD, PFHB1, IVF, HB2, HH1, SSS1, CDCD2, CMPD2, ICCD | 3p21 | M27902, M77235, Q86V90, Q14524, A5H1P8, A5H1P8 |
| SCN7A | sodium channel, voltage-gated, type VII, alpha subunit | astrocytes PNS (DRG) | Nav2.1, Nav2.2, NaG | 2q21-q23 | M96578 Y09164, Q01118 |

(continued)

| Approved Symbol | Approved Name | Tissue distribution | Synonyms | Chromosome | Accession number |
|---|---|---|---|---|---|
| SCN8A | sodium channel, voltage gated, type VIII, alpha subunit | CNS | Nav1.6, NaCh6, PN4, CerIII | 12q13.1 | L39018 AF049239A F049240 U26707 AF049617 AF050736 AF003373 Q9UQD0, Q9UPB2, B9VWG8 |
| SCN9A | sodium channel, voltage-gated, type IX, alpha subunit | medullary thyroid Ca Schwann Cells PNS | Nav1.7, PN1, NE-NA, NENA, ETHA | 2q24 | Q15858 A1BUH5 Q8WWN4,U79568 X82835 U35238 |
| SCN10A | sodium channel, voltage-gated, type X, alpha subunit | PNS | Nav1.8, hPN3, SNS, PN3 | 3p22.2 | Q9Y5Y9, A6NDQ1 , X92184 U53833 Y09108 |
| SCN11A | sodium channel, voltage-gated, type XI, alpha subunit | | Nav1.9, NaN, SNS-2 | 3p22.2 | Q9UI33, A6NN05 Q9UHM0 |

Table 2: Calcium channels, voltage-gated

| Approved Symbol | Approved Name | Tissue distribution | Synonyms | Chromosome | Accession Number |
|---|---|---|---|---|---|
| CACNA1A | calcium channel, voltage-dependent, P/Q type, alpha 1A subunit | Brain specific; mainly found in cerebellum, cerebral cortex, thalamus and hypothalamus. Expressed in the small cell lung carcinoma cell line SCC-9 | Cav2.1, EA2, APCA, HPCA, FHM | 19p13 | O00555, J3KP41, Q9UDC4 |
| CACNA1B | calcium channel, voltage-dependent, N type, alpha 1B subunit | Isoform Alpha-1b-1 and isoform Alpha-lb-2 are expressed in the central nervous system | Cav2.2, CACNN | 9q34 | Q00975, B1AQK5 |
| CACNA1C | calcium channel, voltage-dependent, L type, alpha 1C subunit | Expressed in brain, heart, jejunum, ovary, pancreatic beta-cells and vascular smooth muscle. Overall expression is reduced in atherosclerotic vascular smooth muscle | Cav1.2, CACH2, CACN2, TS, LQT8 | 12p13.3 | Q13936, B2RUT3 Q99875 |

(continued)

| Approved Symbol | Approved Name | Tissue distribution | Synonyms | Chromosome | Accession Number |
|---|---|---|---|---|---|
| CACNA1D | calcium channel, voltage-dependent, L type, alpha 1D subunit | Expressed in pancreatic islets and in brain, where it has been seen in cerebral cortex, hippocampus, basal ganglia, habenula and thalamus. Expressed in the small cell lung carcinoma cell line SCC-9. | Cav1.3, CACH3, CACN4 | 3p14.3 | Q01668, B0FYA3, Q9UDC3 |
| CACNA1E | calcium channel, voltage-dependent, R type, alpha 1E subunit | Expressed in neuronal tissues and in kidney | Cav2.3, BII, CACH6 | 1q25.3 | Q15878, Q14581, B1AM12 |
| CACNA1F | calcium channel, voltage-dependent, L type, alpha 1F subunit | Expression in skeletal muscle and retina | Cav1.4, JM8, JMC8, CSNBX2, CORDX3, CSNB2A, OA2 | Xp11.23 | O60840, A6NI29, Q9UHB1 |
| CACNA1G | calcium channel, voltage-dependent, T type, alpha 1G subunit | Highly expressed in brain, in particular in the amygdala, subthalamic nuclei, cerebellum and thalamus. Moderate expression in heart; low expression in placenta, kidney and lung. Also expressed in colon and bone marrow and in tumoral cells to a lesser extent. Highly expressed in fetal brain, but also in peripheral fetal tissues as heart, kidney and lung, suggesting a developmentally regulated expression | Cav3.1, NBR13 | 17q22 | O43497, D6RA64, Q9Y5T3 |
| CACNA1H | calcium channel, voltage-dependent, T type, alpha 1H subunit | Expressed in kidney, liver, heart, brain. Isoform 2 seems to be testis-specific | Cav3.2 | 16p13.3 | 095180, B5ME00, Q9NYY5 |
| CACNA1I | calcium channel, voltage-dependent, T type, alpha 1I subunit | Brain specific | Cav3.3 | 22q13.1 | Q9P0X4, BOQY12, Q9UNE6 |

(continued)

| Approved Symbol | Approved Name | Tissue distribution | Synonyms | Chromosome | Accession Number |
|---|---|---|---|---|---|
| CACNA1S | calcium channel, voltage-dependent, L type, alpha 1S subunit | Skeletal muscle specific. | Cav1.1, hypoPP | 1q32 | Q13698, A4IF51, Q13934 |

Table 3: Transient receptor potential cation channels

| Approved Symbol | Approved Name | Tissue distribution | Synonyms | Chromosome | Accession number |
|---|---|---|---|---|---|
| TRPA1 | transient receptor potential cation channel, subfamily A, member 1 | Expressed at very low level in human fibroblasts and at a moderate level in liposarcoma cells | | 8q13 | O75762, A6NIN6 |
| TRPC1 | transient receptor potential cation channel, subfamily C, member 1 | Seems to be ubiquitous | HTRP-1 | 3q23 | P48995, Q14CE4 |
| TRPC2 | transient receptor potential cation channel, subfamily C, member 2, pseudogene | | | 11p15.4 | Q6ZNB5 |
| TRPC3 | transient receptor potential cation channel, subfamily C, member 3 | Expressed predominantly in brain and at much lower levels in ovary, colon, small intestine, lung, prostate, placenta and testis | | 4q27 | Q13507, A7VJS1, Q5G1L5 |
| TRPC4 | transient receptor potential cation channel, subfamily C, member 4 | Strongly expressed in placenta. Expressed at lower levels in heart, pancreas, kidney and brain. Expressed in endothelial cells. Isoform alpha was found to be the predominant isoform. | HTRP4, TRP4 | 13q13.3 | Q9UBN4, B1ALE0, Q9UIB2 |

(continued)

| Approved Symbol | Approved Name | Tissue distribution | Synonyms | Chromosome | Accession number |
|---|---|---|---|---|---|
| TRPC5 | transient receptor potential cation channel, subfamily C, member 5 | Expressed in brain with higher levels in fetal brain. Found in cerebellum and occipital pole | PPP1R159 | Xq23 | Q9UL62, B2RP53 Q9Y514 |
| TRPC6 | transient receptor potential cation channel, subfamily C, member 6 | Expressed primarily in placenta, lung, spleen, ovary and small intestine. Expressed in podocytes and is a component of the glomerular slit diaphragm | TRP6 | 11q22.1 | Q9Y210 Q52M59 Q9NQA9 |
| TRPC7 | transient receptor potential cation channel, subfamily C, member 7 | Highly expressed in brain and peripheral blood cells, such as neutrophils. Also detected in bone marrow, spleen, heart, liver and lung. Isoform 2 is found in neutrophil granulocytes | | 5q31.2 | 094759 D3DSL6 Q96Q93_Q9HCX4 A1A4Z4 Q8IWP7 |
| TRPM1 | transient receptor potential cation channel, subfamily M, member 1 | Expressed in the retina where it localizes to the outer plexiform layer. Highly expressed in benign melanocytic nevi and diffusely expressed in various in situ melanomas, but not detected in melanoma metastases. Also expressed in melanocytes and pigmented metastatic melanoma cell lines. In melanocytes expression appears to be regulated at the level of transcription and mRNA processing | LTRPC1, CSNB1C | 15q13.3 | Q7Z4N2 D9IDV2 Q7Z4N5 |
| TRPM2 | transient receptor potential cation channel, subfamily M, member 2 | Detected in blood plasma, cerebrospinal fluid, milk, seminal plasma and colon mucosa. Detected in the germinal center of colon lymphoid nodules and in colon parasympathetic ganglia of the Auerbach plexus (at protein level). Ubiquitous. Detected in brain, testis, ovary, liver and pancreas, and at lower levels in kidney, heart, spleen and lung | KNP3, LTRPC2, NUDT9L1, NUDT9H, EREG1 | 21q22.3 | P10909 B2R9Q1 Q7Z5B9 |

| Approved Symbol | Approved Name | Tissue distribution | Synonyms | Chromosome | Accession number |
|---|---|---|---|---|---|
| TRPM3 | transient receptor potential cation channel, subfamily M, member 3 | | KIAA1616, LTRPC3, GON-2 | 9q21.11 | Q504Y1 |
| TRPM4 | transient receptor potential cation channel, subfamily M, member 4 | Widely expressed with a high expression in intestine and prostate. In brain, it is both expressed in whole cerebral arteries and isolated vascular smooth muscle cells. Prominently expressed in Purkinje fibers. Expressed at higher levels in T-helper 2 (Th2) cells as compared to T-helper 1 (Th1) cells | FLJ20041 | 19q13.3 | Q8TD43 A2RU25 Q9NXV1 |
| TRPM5 | transient receptor potential cation channel, subfamily M, member 5 | Strongly expressed in fetal brain, liver and kidney, and in adult prostate, testis, ovary, colon and peripheral blood leukocytes. Also expressed in a large proportion of Wilms' tumors and rhabdomyosarcomas. In monochromosomal cell lines shows exclusive paternal expression | LTRPC5, MTR1 | 11p15.5 | Q9NZQ8 A6NHS0, Q52LU2, Q9NY34 |
| TRPM6 | transient receptor potential cation channel, subfamily M, member 6 | Highly expressed in kidney and colon. Isoform TRPM6a and isoform TRPM6b, are coexpressed with TRPM7 in kidney, and testis, and are also found in several cell lines of lung origin. Isoform TRPM6c is detected only in testis and in NCI-H5 10A small cell lung carcinoma cells. | CHAK2, FLJ22628 | 9q21.13 | Q9BX84 Q6VPR8 Q6VPS2 |
| TRPM7 | transient receptor potential cation channel, subfamily M, member 7 | | CHAK1, LTRPC7, TRP-PLIK | 15q21 | Q96QT4 Q6ZMF5 Q9NXQ2 |

(continued)

| Approved Symbol | Approved Name | Tissue distribution | Synonyms | Chromosome | Accession number |
|---|---|---|---|---|---|
| TRPM8 | transient receptor potential cation channel, subfamily M, member 8 | Expressed in prostate. Also expressed in prostate tumors and in non-prostatic primary tumors such as colon, lung, breast and skin tumors | | 2q37 | Q7Z2W7, A0AVG2, Q9BVK1 |
| MCOLN1 | mucolipin 1 | Widely expressed in adult and fetal tissues | TRPML1, ML4, MLIV, MST080, MSTP080, TRPM-L1 | 19p13.2 | Q9GZU1 D6W647 Q9H4B5 |
| MCOLN2 | mucolipin 2 | | TRPML2, FLJ36691, TRP-ML2 | 1p22 | Q8IZK6 A6NI99 Q8N9R3 |
| MCOLN3 | mucolipin 3 | | TRPML3, FLJ11006, TRP-ML3 | 1p22.3 | Q8TDD5 Q5T4H5, Q5T4H6, Q9NV09 |
| PKD1 | polycystic kidney disease 1 (autosomal dominant) | | PBP, Pc-1, TRPP1 | 16p13.3 | P98161 Q15140 Q15141 |
| PKD2 | polycystic kidney disease 2 (autosomal dominant) | Strongly expressed in ovary, fetal and adult kidney, testis, and small intestine. Not detected in peripheral leukocytes | PKD4, PC2, Pc-2, TRPP2 | 4q22.1 | Q13563 O60441 Q2M1Q5 |
| PKD2L1 | polycystic kidney disease 2-like 1 | Expressed in adult heart, skeletal muscle, brain, spleen, testis, retina and liver. | PCL, TRPP3 | 10q24.31 | Q9P0L9 O75972 Q9UPA2 |
| PKD2L2 | polycystic kidney disease 2-like 2 | Testis, Brain and Kidney | TRPP5 | 5q31 | Q9NZM6 A6NK98 Q9UNJ0 |

(continued)

| Approved Symbol | Approved Name | Tissue distribution | Synonyms | Chromosome | Accession number |
|---|---|---|---|---|---|
| TRPV1 | transient receptor potential cation channel, subfamily V, member 1 | Widely expressed at low levels. Expression is elevated in dorsal root ganglia. In skin, expressed in cutaneous sensory nerve fibers, mast cells, epidermal keratinocytes, dermal blood vessels, the inner root sheet and the infundibulum of hair follicles, differentiated sebocytes, sweat gland ducts, and the secretory portion of eccrine sweat glands (at protein level). | | 17p13.2 | Q8NER1 A2RUA9 Q9NY22 |
| TRPV2 | transient receptor potential cation channel, subfamily V, member 2 | | VRL, VRL-1, VRL1 | 17p11.2 | Q9Y5S1, A6NML2, A8K0Z0, Q9Y670 |
| TRPV3 | transient receptor potential cation channel, subfamily V, member 3 | Abundantly expressed in CNS. Widely expressed at low levels. Detected in dorsal root ganglion (at protein level). Expressed in the keratinocyte layers of the outer root sheath and, to lesser extent, to the matrix of the hair follicles (at protein level). | VRL3 | 17p13.3 | Q8NET8, Q8NDW7, Q8NET9, Q8NFH2 |
| TRPV4 | transient receptor potential cation channel, subfamily V, member 4 | Found in the synoviocytes from patients with (RA) and without (CTR) rheumatoid arthritis (at protein level). | OTRPC4, TRP12, VROAC, VRL-2, VROAC, CMT2C | 12q24.1 | Q9HBA0, B7ZKQ6,Q9HBC0 |
| TRPV5 | transient receptor potential cation channel, subfamily V, member 5 | Expressed at high levels in kidney, small intestine and pancreas, and at lower levels in testis, prostate, placenta, brain, colon and rectum | CaT2 | 7q34 | Q9NQA5, A4D2H7, Q96PM6 |

(continued)

| Approved Symbol | Approved Name | Tissue distribution | Synonyms | Chromosome | Accession number |
|---|---|---|---|---|---|
| TRPV6 | transient receptor potential cation channel, subfamily V, member 6 | Expressed at high levels in the gastrointestinal tract, including esophagus, stomach, duodenum, jejunum, ileum and colon, and in pancreas, placenta, prostate and salivary gland. Expressed at moderate levels in liver, kidney and testis. Expressed in locally advanced prostate cancer, metastatic and androgen-insensitive prostatic lesions but not detected in healthy prostate tissue and benign prostatic hyperplasia | CaT1 | 7q34 | Q9H1D0, A4D2I8, Q9H296 |

Table 4: CatSper channels

| Approved Symbol | Approved Name | Tissue distribution | Synonyms | Chromosome | Accession number |
|---|---|---|---|---|---|
| CATSPER1 | cation channel, sperm associated 1 | Testis-specific | CATSPER | 11q12.1 | Q8NEC5 Q96P76 |
| CATSPER2 | cation channel, sperm associated 2 | Testis-specific | | 15q15.3 | Q96P56, Q8NHT9, Q96P54, Q96P55 |
| CATSPER3 | cation channel, sperm associated 3 | Testis-specific | CACRC | 5q31.2 | Q86XQ3, Q86XS6 |
| CATSPER4 | cation channel, sperm associated 4 | Testis-specific | | 1p35.3 | Q7RTX7, A1A4W6, Q5VY71 |

Table 5: Two-pore channels

| Approved Symbol | Approved Name | Tissue distribution | Synonyms | Chromosome | Accession number |
|---|---|---|---|---|---|
| TPCN1 | two pore segment channel 1 | | KIAA1169, FLJ20612, TPC1 | 12q24.21 | B7Z3R2 A5PKY2 H0YIK4 F8VV93 |
| TPCN2 | two pore segment channel 2 | Widely expressed. Expressed at high level in liver and kidney | TPC2 | 11q13.1 | Q8NHX9 Q9NT82_E7ETX0 |

Table 6: Cyclic nucleotide-regulated channels

| Approved Symbol | Approved Name | Tissue Distribution | Synonyms | Chromosome | Accession number |
|---|---|---|---|---|---|
| CNGA1 | cyclic nucleotide gated channel alpha 1 | Rod cells in the retina | RCNC1, RCNCa, CNG1, RP49 | 4p12 | P29973 A8K7K6 Q4W5E, D6RCF1_D6R978, A0A024R9X3,Q14028, H3BN09 Q9UMG2 |
| CNGA2 | cyclic nucleotide gated channel alpha 2 | | CNG2, OCNC1, OCNCa, OCNCALPHA, OCNCalpha, FLJ46312 | Xq27 | Q16280,A0AVD0,Q8IV77 |
| CNGA3 | cyclic nucleotide gated channel alpha 3 | Prominently expressed in retina | CCNC1, CCNCa, CNG3 | 2q11.2 | Q16281, Q4VAP7, Q53RD2, Q9UP64 |
| CNGA4 | cyclic nucleotide gated channel alpha 4 | | OCNC2, OCNCb, CNG5 | 11p15.4 | Q8IV77 |
| CNGB1 | cyclic nucleotide gated channel beta 1 | | RCNC2, RCNCb, GARP, GAR1, CNGB1B, RP45 | 16q13 | Q16280,Q16280 |
| CNGB3 | cyclic nucleotide gated channel beta 3 | Expressed specifically in the retina | | 8q21.3 | Q9NQW8 C9JA51, Q9NRE9, H0YAZ4_B9EK43,_QOQD47 |
| HCN1 | hyperpolarization activated cyclic nucleotide-gated potassium channel 1 | Detected in brain, in particular in amygdala and hippocampus, while expression in caudate nucleus, corpus callosum, substantia nigra, subthalamic nucleus and thalamus is very low or not detectable. Detected at very low levels in muscle and pancreas | BCNG-1, HAC-2 | 5p12 | 060741, Q86WJ6 |
| HCN2 | hyperpolarization activated cyclic nucleotide-gated potassium channel 2 | Highly expressed throughout the brain. Detected at low levels in heart. | BCNG-2, HAC-1 | 19p13 | Q9UL51, 060742, Q9UBS2 |
| HCN3 | hyperpolarization activated cyclic nucleotide-gated potassium channel 3 | Detected in brain | KIAA1535 | 1q21.2 | Q9P1Z3, D3DV90, Q9BWQ2, Q2T9L6 Q86WJ5 |

(continued)

| Approved Symbol | Approved Name | Tissue Distribution | Synonyms | Chromosome | Accession number |
|---|---|---|---|---|---|
| HCN4 | hyperpolarization activated cyclic nucleotide-gated potassium channel 4 | Highly expressed in thalamus, testis and in heart, both in ventricle and atrium. Detected at much lower levels in amygdala, substantia nigra, cerebellum and hippocampus | | 15q24.1 | Q9Y3Q4 Q9UMQ7 |

Table 7: Potassium channels, calcium-activated

| Approved Symbol | Approved Name | Tissue distribution | Synonyms | Chromosome | Accession Number |
|---|---|---|---|---|---|
| KCNMA1 | potassium large conductance calcium-activated channel, subfamily M, alpha member 1 | Widely expressed. Except in myocytes, it is almost ubiquitously expressed | KCa1.1, mSLO1 | 10q22 | Q12791 F8WA96 Q9UQK6 |
| KCNN1 | potassium intermediate/ small conductance calcium-activated channel, subfamily N, member 1 | | KCa2.1, hSK1 | 19p13.1 | Q92952 Q5KR10, Q6DJU4 |
| KCNN2 | potassium intermediate/ small conductance calcium-activated channel, subfamily N, member 2 | Expressed in atrial myocytes (at protein level). Widely expressed | KCa2.2, hSK2 | 5q22.3 | Q9H2S1 A6NF94 Q6X2Y2 |
| KCNN3 | potassium intermediate/ small conductance calcium-activated channel, subfamily N, member 3 | | KCa2.3, hSK3, SKCA3 | 1q21.3 | Q9UGI6 B1ANX0 Q8WXG7 |
| KCNN4 | potassium intermediate/ small conductance calcium-activated channel, subfamily N, member 4 | Widely expressed in non-excitable tissues | KCa3.1, hSK4, hKCa4, hIKCa1 | 19q13.2 | 015554 Q53XR4 |
| KCNT1 | potassium channel, subfamily T, member 1 | Highest expression in liver, brain and spinal cord. Lowest .. expression in skeletal muscle | KCa4.1, KIAA1422 | 9q34.3 | Q5JUK3 B3KXF7, Q9P2C5 |
| KCNT2 | potassium channel, subfamily T, member 2 | | KCa4.2, SLICK, SLO2.1 | Iq31.3 | Q6UVM3, Q3SY59, Q5VTN1, Q6ZMT3 |
| KCNU1 | potassium channel, subfamily U, member 1 | Testis-specific | KCa5.1, Slo3, KCNMC1, Kcnma3 | 8p11.2 | A8MYU2 |

Table 8: Potassium channels, voltage-gated

| Approved Symbol | Approved Name | Tissue distribution | Synonyms | Chromosome | Accession number |
|---|---|---|---|---|---|
| KCNA1 | potassium voltage-gated channel, shaker-related subfamily, member 1 (episodic ataxia with myokymia) | | Kv1.1, RBK1, HUK1, MBK1 | 12p13 | Q09470 A6NM83, Q3MIQ9 |
| KCNA2 | potassium voltage-gated channel, shaker-related subfamily, member 2 | | Kv1.2, HK4 | 1p13 | P16389 Q86XG6 |
| KCNA3 | potassium voltage-gated channel, shaker-related subfamily, member 3 | | Kv1.3, MK3, HLK3, HPCN3 | 1p13.3 | P22001 Q5VWN2 |
| KCNA4 | potassium voltage-gated channel, shaker-related subfamily, member 4 | | Kv1.4, HK1, HPCN2 | 11p14 | P22459 |
| KCNA5 | potassium voltage-gated channel, shaker-related subfamily, member 5 | Pancreatic islets and insulinoma | Kv1.5, HK2, HPCN1 | 12p13 | P22460 Q4KKT8 Q9UDA4 |
| KCNA6 | potassium voltage-gated channel, shaker-related subfamily, member 6 | | Kv1.6, HBK2, PPP1R96 | 12p13 | P17658 |

| Approved Symbol | Approved Name | Tissue distribution | Synonyms | Chromosome | Accession number |
|---|---|---|---|---|---|
| KCNA7 | potassium voltage-gated channel, shaker-related subfamily, member 7 | Highly expressed in skeletal muscle, heart and kidney. | Kv1.7, HAK6 | 19q13.3 | Q96RP8, A1KYX7, Q9BYS4 |
| KCNA10 | potassium voltage-gated channel, shaker-related subfamily, member 10 | Detected in kidney, in proximal tubules, glomerular endothelium, in vascular endothelium and in smooth muscle cells | Kv1.8 | 1p13.1 | Q16322 |
| KCNB1 | potassium voltage-gated channel, Shab-related subfamily, member 1 | | Kv2.1 | 20q13.2 | Q14721 Q14193 |
| KCNB2 | potassium voltage-gated channel, Shab-related subfamily, member 2 | | Kv2.2 | 8q13.2 | Q92953, Q7Z7D0, Q9BXD3 |
| KCNC1 | potassium voltage-gated channel, Shaw-related subfamily, member 1 | | Kv3.1 | 11p15 | P48547, K4DI87 |
| KCNC2 | potassium voltage-gated channel, Shaw-related subfamily, member 2 | | Kv3.2 | 12q14.1 | Q96PR1, B7Z231, Q96PR0 |

(continued)

| Approved Symbol | Approved Name | Tissue distribution | Synonyms | Chromosome | Accession number |
|---|---|---|---|---|---|
| KCNC3 | potassium voltage-gated channel, Shaw-related subfamily, member 3 | | Kv3.3 | 19q133 3 | Q14003 |
| KCNC4 | potassium voltage-gated channel, Shaw-related subfamily, member 4 | | Kv3.4, HKSHIIIC | 1p21 | Q03721, Q3MIM4, Q5TBI6 |
| KCND1 | potassium voltage-gated channel, Shal-related subfamily, member 1 | Widely expressed. Highly expressed in brain, in particular in cerebellum and thalamus; detected at lower levels in the other parts of the brain | Kv4.1 | Xp11.23 | Q9NSA2 B2RCG0, O75671 |
| KCND2 | potassium voltage-gated channel, Shal-related subfamily, member 2 | Highly expressed throughout the brain. Expression is very low or absent in other tissues. | Kv4.2, RK5, KIAA1044 | 7q31 | Q9NZV8, 095012 Q9UNH9 |
| KCND3 | potassium voltage-gated channel, Shal-related subfamily, member 3 | Highly expressed in heart and brain, in particular in cortex, cerebellum, amygdala and caudate nucleus. Detected at lower levels in liver, skeletal muscle, kidney and pancreas. Isoform 1 predominates in most tissues. Isoform 1 and isoform 2 are detected at similar levels in brain, skeletal muscle and pancreas | Kv4.3, KSHIVB | 1p13.2 | Q9UK17, O60576, Q9UK16 |
| KCNF1 | potassium voltage-gated channel, subfamily F, member 1 | Detected in heart, brain, liver, skeletal muscle, kidney and pancreas. | Kv5.1, kH1, IK8 | 2p25 | Q9H3M0, 043527, Q585L3 |

(continued)

| Approved Symbol | Approved Name | Tissue distribution | Synonyms | Chromosome | Accession number |
|---|---|---|---|---|---|
| KCNG1 | potassium voltage-gated channel, subfamily G, member 1 | Detected in brain and placenta, and at much lower levels in kidney and pancreas | Kv6.1, kH2, K13 | 20q13 | Q9UIX4, A8K3S4, Q9BRC1 |
| KCNG2 | potassium voltage-gated channel, subfamily G, member 2 | Highly expressed in heart, liver, skeletal muscle, kidney and pancreas. Detected at low levels in brain, lung and placenta | Kv6.2, KCNF2 | 18q23 | Q9UJ96 |
| KCNG3 | potassium voltage-gated channel, subfamily G, member 3 | Detected in many parts of the brain with the exception of the cerebellum, in testis, pancreas, lung, kidney, ovary, small intestine, colon, thymus, adrenal gland and spinal cord | Kv6.3 | 2p21 | Q8TAE7, Q53SC1 |
| KCNG4 | potassium voltage-gated channel, subfamily G, member 4 | Highly expressed in brain, and at lower levels in liver, small intestine and colon. | Kv6.4 | 16q24.1 | Q8TDN1, Q96H24 |
| KCNH1 | potassium voltage-gated channel, subfamily H (eag-related), member 1 | Highly expressed in brain and in myoblasts at the onset of fusion, but not in other tissues. Detected in HeLa (cervical carcinoma), SH-SY5Y (neuroblastoma) and MCF-7 (epithelial tumor) cells, but not in normal epithelial cells. | Kv10.1, eag, h-eag, eag1 | 1q32.2 | **O95259** B1AQ26, O76035, Q14CL3 |
| KCNH2 | potassium voltage-gated channel, subfamily H (eag-related), member 2 | Highly expressed in heart and brain. Isoforms USO are frequently overexpressed in cancer cells | Kv11.1, HERG, erg1 | 7q36.1 | Q12809 A5H1P7 Q9H3P0 |
| KCNH3 | potassium voltage-gated channel, subfamily H (eag-related), member 3 | Detected only in brain, in particular in the telencephalon. Detected in the cerebral cortex, occipital pole, frontal and temporal lobe, putamen, amygdala, hippocampus and caudate nucleus | Kv.12.2, BEC1, | 12q13 | Q9ULD8 Q9UQ06 |

(continued)

| Approved Symbol | Approved Name | Tissue distribution | Synonyms | Chromosome | Accession number |
|---|---|---|---|---|---|
| KCNH4 | potassium voltage-gated channel, subfamily H (eag-related), member 4 | Detected only in brain, in particular in the telencephalon. Detected in putamen and caudate nucleus, and at lower levels in cerebral cortex, occipital and hippocampus | Kv12.3, elk1 | 17q21 | Q9UQ05 |
| KCNH5 | potassium voltage-gated channel, subfamily H (eag-related), member 5 | Detected in brain, skeletal muscle, heart, placenta, lung and liver, and at low levels in kidney | Kv10.2, H-EAG2, eag2 | 14q23.1 | Q8NCM2, C9JP98 |
| KCNH6 | potassium voltage-gated channel, subfamily H (eag-related), member 6 | Expressed in prolactin-secreting adenomas | Kv 11.2, erg2, HERG2 | 17q23.3 | Q9H252, Q9BRD7 |
| KCNH7 | potassium voltage-gated channel, subfamily H (eag-related), member 7 | Expressed in prolactin-secreting adenomas | Kv11.3, HERG3, erg3 | 2q24.3 | Q9NS40, Q53QU4, Q8IV15 |
| KCNH8 | potassium voltage-gated channel, subfamily H (eag-related), member 8 | Nervous system | Kv12.1, elk3 | 3p24.3 | Q96L42, Q59GQ6 |
| KCNQ1 | potassium voltage-gated channel, KQT-like subfamily, member 1 | Abundantly expressed in heart, pancreas, prostate, kidney, small intestine and peripheral blood leukocytes. Less abundant in placenta, lung, spleen, colon, thymus, testis and ovaries. | Kv7.1, KCNA8, KVLQT1, JLNS1, LQT1 | 11p15.5 | P51787, O00347  Q9UMN9 |

(continued)

| Approved Symbol | Approved Name | Tissue distribution | Synonyms | Chromosome | Accession number |
|---|---|---|---|---|---|
| KCNQ2 | potassium voltage-gated channel, KQT-like subfamily, member 2 | In adult and fetal brain. Highly expressed in areas containing neuronal cell bodies, low in spinal chord and corpus callosum. Isoform 2 is preferentially expressed in differentiated neurons. Isoform 6 is prominent in fetal brain, undifferentiated neuroblastoma cells and brain tumors | Kv7.2, ENB1, BFNC, KCNA11, HNSPC | 20q13.33 | 043526, 043796, Q99454 |
| KCNQ3 | potassium voltage-gated channel, KQT-like subfamily, member 3 | Brain | Kv7.3 | 8q24 | O43525, A2VCT8, B4DJY4, E7EQ89 |
| KCNQ4 | potassium voltage-gated channel, KQT-like subfamily, member 4 | Expressed in the outer, but not the inner, sensory hair cells of the cochlea. Slightly expressed in heart, brain and skeletal muscle. | Kv7.4 | 1p34 | P56696, 096025 |
| KCNQ5 | potassium voltage-gated channel, KQT-like subfamily, member 5 | Strongly expressed in brain and skeletal muscle. In brain, expressed in cerebral cortex, occipital pole, frontal lobe and temporal lobe. Lower levels in hippocampus and putamen. Low to undetectable levels in medulla, cerebellum and thalamus. | Kv7.5 | 6q14 | Q9NR82, B4DS33. Q9NYA6 |
| KCNS1 | potassium voltage-gated channel, delayed-rectifier, subfamily S, member 1 | Detected in all tissues tested with the exception of skeletal muscle. Highly expressed in adult and fetal brain, fetal kidney and lung, and adult prostate and testis. | Kv9.1 | 20q12 | A2RUL8,_ Q96KK3, A2RUL9, Q6DJU6, Q92953, Q7Z7D0, Q9BXD3 |
| KCNS2 | potassium voltage-gated channel, delayed-rectifier, subfamily S, member 2 | | Kv9.2 | 8q22 | Q9ULS6, A8KAN1, Q92953, Q7Z7D0, Q9BXD3,_ Q14721, Q14193 |

(continued)

| Approved Symbol | Approved Name | Tissue distribution | Synonyms | Chromosome | Accession number |
|---|---|---|---|---|---|
| KCNS3 | potassium voltage-gated channel, delayed-rectifier, subfamily S, member 3 | Detected in whole normal term placental and placental chorionic plate arteries and veins. Detected in syncytiotrophobl ast and in blood vessel endothelium within intermediate villi and chorionic plate (at protein level). Detected in most tissues, but not in peripheral blood lymphocytes. The highest levels of expression are in lung | Kv9.3 | 2p24 | Q9BQ31, D6W520 Q96B56,_ C9J187, L8E8W4,_ Q92953, Q7Z7D0, Q9BXD3 Q14721, Q14193 |
| KCNV1 | potassium channel, subfamily V, member 1 | Detected in brain | Kv8.1 | 8q23.2 | Q6PIU1, Q9UHJ4, B2R8Q7,_ B4DMC1 Q76FP2 |
| KCNV2 | potassium channel, subfamily V, member 2 | Detected in heart, brain, liver, skeletal muscle, spleen, thymus, prostate, testis, ovary colon, kidney and pancreas | Kv8.2 | 9p24.2 | Q8TDN2, Q5T6X0 P48547, K4DI87,_ Q14721, Q14193,_ Q9H3M0 043527, Q585L3 |

Table 9: Potassium channels, inwardly rectifying

| Approved Symbol | Approved Name | Tissue distribution | Synonyms | Chromosome | Accession number |
|---|---|---|---|---|---|
| KCNJ1 | potassium inwardly-rectifying channel, subfamily J, member 1 | In the kidney and pancreatic islets. Lower levels in skeletal muscle, pancreas, spleen, brain, heart and liver | Kir1.1, ROMK1 | 11q24 | P48048, B2RMR4, Q6LD67, A0A024R3K6 |
| KCNJ2 | potassium inwardly-rectifying channel, subfamily J, member 2 | Heart, brain, placenta, lung, skeletal muscle, and kidney. Diffusely distributed throughout the brain. | Kir2.1, IRK1, LQT7 | 17q24.3 | P63252, 015110, P48049, Q9NPI9 |
| KCNJ3 | potassium inwardly-rectifying channel, subfamily J, member 3 | | Kir3.1, GIRK1, KGA | 2q24.1 | P48549, B4DEW7, Q8TBI0, D2XBF0, D2X9V0 |

(continued)

| Approved Symbol | Approved Name | Tissue distribution | Synonyms | Chromosome | Accession number |
|---|---|---|---|---|---|
| KCNJ4 | potassium inwardly-rectifying channel, subfamily J, member 4 | Heart, skeletal muscle, and several different brain regions including the hippocampus | Kir2.3, HIR, HRK1, hIRK2, IRK3 | 22q13.1 | P48050, Q14D44, A0A024R1L8, P63252, O15110, P48049 |
| KCNJ5 | potassium inwardly-rectifying channel, subfamily J, member 5 | Islets, exocrine pancreas and heart. Expressed in the adrenal cortex, particularly the zona glomerulosa | Kir3.4, CIR, KATP1, GIRK4, LQT13 | 11q24 | Q4QRJ2,_P48544, B2R744 Q92807, H9A8K9, H7CGH0, A0A024R3K7 |
| KCNJ6 | potassium inwardly-rectifying channel, subfamily J, member 6 | Widely expressed. Expressed in cells of hematopoietic origin (at protein level). Most abundant in cerebellum, and to a lesser degree in islets and exocrine pancreas | Kir3.2, GIRK2, KATP2, BIR1, hiGIRK2 | 21q22.1 | P48051 Q3MJ74, Q53WW6,_ B2RA12, Q96L92, Q32Q36 Q9H3K8 |
| KCNJ8 | potassium inwardly-rectifying channel, subfamily J, member 8 | Predominantly detected in fetal and adult heart | Kir6.1 | 12p12.1 | Q15842, O00657, F5GY12, A0A024RAV6 |
| KCNJ9 | potassium inwardly-rectifying channel, subfamily J, member 9 | Widely expressed. Expressed in cells of hematopoietic origin (at protein level). | Kir3.3, GIRK3 | 1q23.2 | Q92806, Q5JW75, Q96L92, Q32Q36, Q9H3K8 |
| KCNJ10 | potassium inwardly-rectifying channel, subfamily J, member 10 | | Kir4.1, Kir1.2 | 1q23.2 | P78508, A3KME7,Q92808, Q547K1, Q9BXC5 Q9NPI9 |
| KCNJ11 | potassium inwardly-rectifying channel, subfamily J, member 11 | | Kir6.2, BIR | 11p15.1 | Q14654, B4DWI4,Q8IW96 E9PPF1, H0YES9, D2K1F9 |
| KCNJ12 | potassium inwardly-rectifying channel, subfamily J, member 12 | Heart, skeletal muscle, and several different brain regions including the hippocampus | Kir2.2, Kir2.2v, IRK2, hIRK1 | 17p11.1 | Q14500, O43401, Q15756, Q8NG63, B7U540, Q9HAP6, P48050, Q14D44 |

(continued)

| Approved Symbol | Approved Name | Tissue distribution | Synonyms | Chromosome | Accession number |
|---|---|---|---|---|---|
| KCNJ13 | potassium inwardly-rectifying channel, subfamily J, member 13 | Predominantly expressed in small intestine. Expression is also detected in kidney, and all central nervous system regions tested with the exception of spinal cord | stomach, Kir7.1, Kir1.4, LCA16 | 2q37 | O60928 A0PGH1 Q8N3Y4_C9JWD6 H7C4D1 |
| KCNJ14 | potassium inwardly-rectifying channel, subfamily J, member 14 | Expressed preferentially in retina | Kir2.4, IRK4 | 19q13 | Q9UNX9 Q99712 D3DSH5 Q99446 |
| KCNJ15 | potassium inwardly-rectifying channel, subfamily J, member 15 | | Kir4.2, Kir1.3, IRKK | 21q22.2 | Q99712 D3DSH5 Q99446, A8K9U7 |
| KCNJ16 | potassium inwardly-rectifying channel, subfamily J, member 16 | Highly expressed in kidney, pancreas and thyroid gland | Kir5.1, BIR9 | 17q24.3 | Q9NPI9 |
| KCNJ18 | potassium inwardly-rectifying channel, subfamily J, member 18 | Specifically expressed in skeletal muscle | KIR2.6, TTPP2 | 17p11.2 | B7U540 |

Table 10: Potassium channels, two-P

| Approved Symbol | Approved Name | Tissue distribution | Synonyms | Chromosome | Accession number |
|---|---|---|---|---|---|
| KCNK1 | potassium channel, subfamily K, member 1 | Widely expressed with high levels in heart and brain and lower levels in placenta, lung, liver and kidney | K2p1.1, DPK, TWIK-1 | 1q42-q43 | 000180, Q13307, Q5T5E8 |
| KCNK2 | potassium channel, subfamily K, member 2 | | K2p2.1, TREK-1 | 1q41 | Q6ZW95, Q13303, A0AVM9, Q99411 |

(continued)

| Approved Symbol | Approved Name | Tissue distribution | Synonyms | Chromosome | Accession number |
|---|---|---|---|---|---|
| KCNK3 | potassium channel, subfamily K, member 3 | Widespread expression in adult. Strongest expression in pancreas and placenta. Lower expression in brain, lung, prostate, heart, kidney, uterus, small intestine and colon | K2p3.1, TASK, TASK-1 | 2p23 | O14649, Q53SU2, B9EIJ4 |
| KCNK4 | potassium channel, subfamily K, member 4 | | K2p4.1, TRAAK | 11q13 | Q2YDA1 |
| KCNK5 | potassium channel, subfamily K, member 5 | Abundant expression in kidney, also detected in liver, placenta and small intestine. In the kidney, expression is restricted to the distal tubules and collecting ducts. | K2p5.1, TASK-2 | 6p21 | O95279 B2RAQ6, B5TJL2, Q5VV76 |
| KCNK6 | potassium channel, subfamily K, member 6 | Widespread expression, detected in all tissues tested except for skeletal muscle. Strongest expression in placenta, pancreas, heart, colon and spleen, lower levels detected in peripheral blood leukocytes, lung, liver, kidney and thymus. Lowest expression detected in brain | K2p6.1, TWIK-2 | 19q13.1 | Q9Y257 Q9HB47 |
| KCNK7 | potassium channel, subfamily K, member 7 | | K2p7.1 | 11q13 | Q3MI97 |
| KCNK9 | potassium channel, subfamily K, member 9 | Mainly found in the cerebellum. Also found in adrenal gland, kidney and | K2p9.1, TASK3, TASK-3 | 8q24.3 | Q9NPC2, Q2M290, Q540F2 |
| KCNK10 | potassium channel, subfamily K, member 10 | Abundantly expressed in pancreas and kidney and to a lower level in brain, testis, colon, and small intestine. Isoform b is strongly expressed in kidney (primarily in the proximal tubule) and pancreas, whereas isoform c is abundantly expressed in brain | K2p 10.1, TREK-2, TREK2, PPP1R97 | 14q31 | P57789, B2R8T4, Q9HB59 |
| KCNK12 | potassium channel, subfamily K, member 12 | | THIK-2, THIK2, K2p12.1 | 2p16.3 | Q9HB15 |

(continued)

| Approved Symbol | Approved Name | Tissue distribution | Synonyms | Chromosome | Accession number |
|---|---|---|---|---|---|
| KCNK13 | potassium channel, subfamily K, member 13 | | K2p13.1, THIK-1, THIK1 | 14q32.11 | Q9HB14, B5TJL8, Q96E79 |
| KCNK15 | potassium channel, subfamily K, member 15 | Detected in pancreas, heart, placenta, lung, liver, kidney, ovary, testis, skeletal muscle and adrenal gland, and at lower levels in prostate, spleen and thyroid gland | K2p15.1, dJ781B1.1, KT3.3, KIAA0237, TASK5, TASK-5 | 20q13.12 | Q9H427, Q52LL3, Q9HBC8 |
| KCNK16 | potassium channel, subfamily K, member 16 | Highly expressed in pancreas. | K2p16.1, TALK-1, TALK1 | 6p21.2-p21.1 | Q96T55, B5TJL9, Q9H591 |
| KCNK17 | potassium channel, subfamily K, member 17 | | K2p17.1, TALK-2, TALK2, TASK4, TASK-4 | 6p21 | Q96T54, E9PB46, Q9H592, B2RCT9 |
| KCNK18 | potassium channel, subfamily K, member 18 | Expressed specifically in dorsal root ganglion and trigeminal ganglion neurons. Detected at low levels in spinal cord. | K2p18.1, TRESK-2, TRESK2, TRESK, TRIK | 10q26.11 | Q7Z418, Q5SQQ8 |

Table 11: Hydrogen voltage-gated ion channels

| Approved Symbol | Approved Name | Tissue distribution | Synonyms | Chromosome | Accession number |
|---|---|---|---|---|---|
| HVCN1 | hydrogen voltage-gated channel 1 | Enriched in immune tissues, such as lymph nodes, B-lymphocytes, monocytes and spleen | MGC15619, Hv1, VSOP | 12q24.11 | Q96D96, A8MQ37, Q96IS5 |

The nucleic acid according to the current invention can encode a portion or entire fragment of voltage-dependent cation channel subunit as elaborated in Tables 1-11 or specifically selected from Calcium-activated potassium channels including subfamily M, alpha member 1, subfamily N, member 1, subfamily N, member 2, subfamily N, member 3, subfamily N, member 4, potassium channel, subfamily T, member 1, potassium channel, subfamily T, member 2 , potassium channel, subfamily U, member 1, CatSper (1 to 4) and Two-Pore channels, Cyclic nucleotide-regulated channels including channel alpha 1, channel alpha 2, channel alpha 3, channel alpha 4 , channel beta 1 , channel beta 3 , hyperpolarization activated cyclic nucleotide-gated potassium channel 1 to 4, potassium channels, Two-P including subfamily K, member 1-18, potassium channels including inwardly rectifying potassium channels like subfamily J, member 1-18, Voltage-gated calcium channels, Voltage-gated potassium channels including shaker-related subfamily, member 1-10, Shab-related subfamily, member 1 and 2, Shaw-related subfamily, member 1 to 4, Shal-related subfamily, member 1-3, subfamily F, member 1, subfamily G, member 1, subfamily G, member 2, subfamily G, member 3, subfamily G, member 4, subfamily H (eag-related), member 1-8, KQT-like subfamily, member 1-5, delayed-rectifier, subfamily S member 1-3, subfamily V, member 1 -2, Voltage-gated sodium channels including type I, alpha subunit, type I, beta subunit, type II, alpha subunit, type II, beta subunit, type III, alpha subunit, type III, beta subunit, type IV, alpha subunit, type V, alpha subunit, type VII, alpha subunit, type VIII, alpha subunit, type IX, alpha subunit, type X, alpha subunit, type XI, alpha subunit, Calcium channel, voltage-dependent including P/Q type, alpha 1A subunit, N type, alpha 1B subunit, L type, alpha 1C subunit, L type, alpha 1D subunit, R type, alpha 1E subunit, L type, alpha 1F subunit, T type, alpha 1G subunit, T type, alpha 1H subunit, T type, alpha 1I subunit, L type, alpha 1S subunit, Transient Receptor Potential channels like Transient receptor potential cation channels including subfamily A, member 1, subfamily C, member 1, subfamily C, member 2, pseudogene, subfamily C, member 3, subfamily C, member 4, subfamily C, member 5, subfamily C, member 6, subfamily C, member 7, subfamily M, member 1, subfamily M, member 2, subfamily M, member 3, subfamily M, member 4, subfamily M, member 5, subfamily M, member 6, subfamily M, member 7, subfamily M, member 8, mucolipin, subfamily V, member 1, subfamily V, member 2, subfamily V, member 3, subfamily V, member 4, subfamily V, member 5, subfamily V, member 6, Hydrogen voltage-gated ion channels and the like.

In preferred embodiments, the voltage-dependent cation channel subunit is a subunit of channel comprising 6 transmembrane domains, 2 intracellular loops, 1 transmembrane loop, and intracellular N- and C-termini, such as transient receptor potential (TRP) channel. Such TRP may be TRPC (canonical) channel, TRPV (vanilloid), TRPM (melastatin), TRPA (ankyrin), TRPP (polycystin), or TRPML (mucolipin).

In particularly preferred embodiments, TRPV channel is TRPV1, TRPV2, TRPV3, TRPV4, TRPV5, or TRPV6, wherein said TRPC channel is TRPC1, TRPC2, TRPC3, TRPC4, TRPC5, TRPC6, or TRPC7, wherein said TRPM channel is TRPM1, TRPM2, TRPM3, TRPM4, TRPM5, TRPM6, TRPM7, or TRPM8, wherein said TRPP channel is TRPP1, TRPP2, TRPP3, or TRPP4, or wherein TRPA is TRPA1.

In particularly preferred embodiments, TRPV channel is TRPV1, TRPV2, TRPV3, TRPV4, or TRPA1.

Embodiments of this disclosure include bioluminescence resonance energy transfer (BRET) systems, methods of detecting a protein-protein interaction, methods to determine efficacy of a test compound, BRET vectors, kits relating to each of the above. In general, BRET systems involve the non-radiative transfer of energy between a bioluminescence donor molecule and a fluorescent acceptor molecule by the FORSTER mechanism. The energy transfer primarily depends on: (i) an overlap between the emission and excitation spectra of the donor and acceptor molecules, respectively and (ii) the proximity of about 100 Angstroms (A) between the donor and acceptor molecules. The donor molecule in BRET produces light via chemiluminescence, so it is amenable to small animal imaging. In addition, the BRET system does not use an external light excitation source, which provides potentially greater sensitivity in living subjects because of the low signal to noise ratio.

An embodiment of a BRET system, among others, includes: nucleic acid comprising a nucleotide sequence encoding a voltage-dependent ion channel fusion subunit bound to a nucleotide sequence encoding at least one bioluminescent donor molecule and/or bound to a nucleotide sequence encoding at least one fluorescent acceptor molecule.

The bioluminescent donor molecule and the fluorescent acceptor molecule are located within specified region of the voltage-dependent ion channel subunits. More precisely, said bioluminescent donor molecule and acceptor molecule may be bound to either C-terminal or N-terminal of said channel subunit, optionally via a linker sequence. Possible coupling according to the present invention may be as follows:

- the bioluminescent donor molecule is bound to C-terminal of channel subunit and acceptor molecule is bound to N-terminal of said channel subunit, or
- the bioluminescent donor molecule is bound to N-terminal of channel subunit and acceptor molecule is bound to C-terminal of said channel subunit.

In highly preferred aspects of the invention, the bioluminescent donor molecule is bound to the C terminus of the channel subunit and the acceptor molecule is bound to the N terminus of the channel subunit.

In preferred aspects of the invention, said bioluminescent donor molecule and acceptor molecule are selected so that the emission spectrum of the bioluminescent donor molecule overlaps with the absorbance spectrum of the acceptor protein, so the light energy delivered by the bioluminescent donor molecule is at a wavelength that is able to excite the acceptor molecule.

Bioluminescent donor refers to any moiety capable of acting on a suitable substrate to transform chemical energy into light energy. For example, it may refer to an enzyme which converts a substrate into an activated product which then releases energy as it relaxes. The activated product (generated by the activity of the bioluminescent protein on the substrate) is the source of the bioluminescent protein-generated luminescence that is transferred to the acceptor molecule.

Given the size of bioluminescent molecules it was surprising that functional voltage-dependent ion channel subunits according to the present invention could be produced. There are a number of different bioluminescent donor molecules that can be employed in the present invention. Light-emitting systems have been known and isolated from many luminescent organisms including bacteria, protozoa, coelenterates, molluscs, fish, millipedes, flies, fungi, worms, crustaceans, and beetles, particularly click beetles of genus Pyrophorus and the fireflies of the genera Photinus, Photuris, and Luciola. Additional organisms displaying bioluminescence are listed in international publications WO 00/024878 and WO 99/049019.

One well characterized example is the class of proteins known as luciferases. Luciferases proteins catalyze an energy-yielding chemical reaction in which a specific biochemical substance, a luciferin (a naturally occurring substrate), is oxidized by an enzyme having a luciferase activity. Both prokaryotic and eukaryotic organisms including species of bacteria, algae, fungi, insects, fish and other marine forms can emit light energy in this manner and each has specific luciferase activities and luciferins, which are chemically distinct from those of other organisms. Luciferin/luciferase systems are very diverse in form, chemistry and function. For example, there are luciferase activities which facilitate continuous chemiluminescence, as exhibited by some bacteria and mushrooms, and those which are adapted to facilitate sporadic, or stimuli induced, emissions, as in the case of dinoflagellate algae. As a phenomenon, which entails the transformation of chemical energy into light energy, bioluminescence is not restricted to living organisms, nor does it require the presence of living organisms. It is simply a type of chemiluminescent reaction that requires a luciferase activity, which at one stage or another had its origins from a biological catalyst. Hence the preservation or construction of the essential activities and chemicals suffices to have the means to give rise to bioluminescent phenomena.

Examples of bioluminescent proteins with luciferase activity may be found in US 5,229,285, 5,219,737, 5,843,746, 5,196,524, and 5,670,356. Two of the most widely used luciferases are: (i) Renilla luciferase (from R. reniformis), a 35 kDa protein, which uses coelenterazine as a substrate and emits light at 480 nm; and (ii) Firefly luciferase (from Photinus pyralis), a 61 kDa protein, which uses luciferin as a substrate and emits light at 560 nm. In preferred embodiments bioluminescent donor molecule is a protein chosen among luciferase, chosen among Renilla luciferase, Firefly luciferase, Coelenterate luciferase, North American glow worm luciferase, click beetle luciferase, a railroad worm luciferase, Gaussia luciferase, Aequorin, Arachnocampa luciferase, or a biologically active variant or fragment of any one. Gaussia luciferase (from Gaussia princeps) is a 20 kDa protein that oxidises coelenterazine in a rapid reaction resulting in a bright light emission at 470 nm. Luciferases useful for the present invention have also been characterized from Anachnocampa sp (WO 2007/019634). These enzymes are about 59 kDa in size and are ATP-dependent luciferases that catalyze luminescence reactions with emission spectra within the blue portion of the spectrum.

Alternative, non-luciferase, bioluminescent molecules may be any enzymes which can act on suitable substrates to generate a luminescent signal. Specific examples of such enzymes are β-galactosidase, lactamase, horseradish peroxydase, alkaline phosphatase, β-glucuronidase, or β-glucosidase. Synthetic luminescent substrates for these enzymes are well known in the art and are commercially available from companies, such as Tropix Inc. (Bedford, MA, USA).

By way of examples, several bioluminescent donor molecules have been listed in the following Table 12.

Table 12: Examples of bioluminescent molecules

| Species | Name | Organism | MW kDa×10⁻³ | Emission (nm) | Substrate |
|---|---|---|---|---|---|
| **Insect** | FFluc | *Photinus pyralis* (North American Firefly) | ~61 | 560 | D-(-)-2-(6'-hydroxyberzothiazolyl)-$\Delta^2$-thiazoline-4-carboxylic acid, HBTTCA, $(C_{11}H_8N_2O_3S_2)$ (Luciferin) |
| **Insect** | FF'luc | *Luciola cruciate* (Japanese Firefly) | | 560-590 (many mutants) | Luciferin |
| **Insect** | | Phengodid beetles (railroad worms) | | | |
| **Insect** | | *Arachnocampa* sp. | | | Luciferin |
| **Insect** | | *Orphelia fultoni* (North American glow worm) | | | |
| **Insect** | Clluc | *Pyrophorus plagiophthalamus* | | 546, 560 578 and 593 | Luciferin |
| **Jelly Fish** | Aequorea | *Aequorea* | 44.9 | 460-470 | Coelenterazine |
| **Sea Pansy** | Rluc | *Renilla Reniformis* | 36 | 480 | Coelenterazine |

(continued)

| Species | Name | Organism | MW kDa×10<sup>-3</sup> | Emission (nm) | Substrate |
|---|---|---|---|---|---|
| Sea Pansy (modified) | Rluc8 | *Renilla Reniformis (modified)* | 36 | 487(peak) | Coelenterazine/Deep Blue C |
| Sea Pansy | Rmluc | *Renilla mullerei* | 36.1 | ~480 | Coelenterazine |
| Sea Pansy | | *Renilla kollikeri* | | | |
| Crustacea (shrimp) | Vluc | *Vargula hilgendorfii* | ~62 | ~460 | coelenterazine* |
| Crustacea | | *Cypridina(sea firefly)* | 75 | 460 | coelenterazine** |
| Dinofagellate (marine algae) | | *Gonyaulax polyedra* | 130 | ~475 | Tetrapyrrole |
| Mollusc | | *Latia (Freshwater limpet)* | 170 | 500 | Enol formate,terpene,aldehyde. |
| Hydroid | | Obelia *biscuspidata* | ~20 | ~470 | Coelenterazine |
| Shrimp | | *Oplophorus gracilorostris* | 31 | 462 | Coelenterazine |
| Others | Ptluc | *Ptilosarcus* | | ~490 | Coelenterazine |
| | Glue | *Gaussia* | ~20 | ~475 | Coelenterazine |
| | Plluc | *Pleuromamma* | 22.6 | ~475 | Coelenterazine |

The choice of the substrate of the bioluminescent donor molecule can impact on the wavelength and the intensity of the light generated by the bioluminescent protein. A widely known substrate is coelenterazine which occurs in cnidarians, copepods, chaetognaths, ctenophores, decapod shrimps, mysid shrimps, radiolarians and some fish taxa. For *Renilla luciferase* for example, coelenterazine analogues/derivatives are available that result in light emission between 418 and 512 nm. A coelenterazine analogue/derivative (400A, DeepBlueC) has been described emitting light at 400 nm with *Renilla luciferase* (WO 01/46691). Other examples of coelenterazine analogues/derivatives are EnduRen and ViviRen.

Luciferin relates to a class of light-emitting biological pigments found in organisms capable of bioluminescence, which are oxidised in the presence of the enzyme luciferase to produce oxyluciferin and energy in the form of light. Luciferin, or 2-(6-hydroxybenzothiazol-2-yl)-2-thiazoline-4-carboxylic acid, was first isolated from the firefly *Photinus pyralis.* Since then, various forms of luciferin have been discovered and studied from various different organisms, mainly from the ocean, for example fish and squid, however, many have been identified for example in worms, beetles and various other insects.

There are at least five general types of luciferin, which are each chemically different and catalysed by chemically and structurally different luciferases that employ a wide range of different cofactors. First, is firefly luciferin, the substrate of firefly luciferase, which requires ATP for catalysis (EC 1.13.12.7). Second, is bacterial luciferin, also found in some squid and fish, that consists of a long chain aldehyde and a reduced riboflavin phosphate. Bacterial luciferase is FMNH-dependent. Third, is dinoflagellate luciferin, a tetrapyrrolic chlorophyll derivative found in dinoflagellates (marine plankton), the organisms responsible for night-time ocean phosphorescence. Dinoflagellate luciferase catalyses the oxidation of dinoflagellate luciferin and consists of three identical and catalytically active domains. Fourth, is the imidazolopyrazine vargulin, which is found in certain ostracods and deep-sea fish, for example, Porichthys. Last, is coelanterazine (an imidazolpyrazine), the light-emitter of the protein aequorin, found in radiolarians, ctenophores, cnidarians, squid, copepods, chaetognaths, fish and shrimp.

There are a number of different acceptor molecules that can be employed in this invention. The acceptor molecule may be a protein or non-proteinaceous.

Representative acceptor proteins can include, but are not limited to, green fluorescent protein (GFP), variant of green fluorescent protein (such as GFP10), blue fluorescent protein (BFP), cyan fluorescent protein (CFP), yellow fluorescent protein (YFP), enhanced GFP (EGFP), enhanced CFP (ECFP), enhanced YFP (EYFP), GFPS65T, mAmetrine, LSS-mOrange, LSS-mKate, Emerald, Topaz, GFPuv, destabilised EGFP (dEGFP), destabilised ECFP (dECFP), destabilised EYFP (dEYFP), HcRed, t-HcRed, DsRed, DsRed2, mRFPl, pocilloporin, *Renilla* GFP, Monster GFP, paGFP, Kaede protein or a Phycobiliprotein, or a biologically active variant or fragment of any one thereof.

The most frequently used bioluminescent or fluorophore is the green fluorescent protein from the jellyfish Aequorea victoria and numerous other variants (GFPs) obtained for example mutagenesis and chimeric protein technologies. GFPs are classified based on the distinctive component of their chromophores, each class having distinct excitation and emission wavelengths: class 1, wild-type mixture of neutral phenol and anionic phenolate: class 2, phenolate anion : class 3, neutral phenol: class 4, phenolate anion with stacked s-electron system: class 5, indole : class 6, imidazole : and class 7, phenyl. One example of an engineered system which is suitable for BRET is a Renilla luciferase and enhanced yellow mutant of GFP (EYFP) pairing which do not directly interact to a significant degree with one another alone in the absence of a mediating protein(s) (in this case, the G protein coupled receptor).

Examples of non-proteinaceous acceptor molecules are Alexa™ (Molecular Probes), fluor dye, Bodipy dye™ (Life technologies), Cy dye™(Life technologies), fluorescein, dansyl, umbelliferone (7-hydroxycoumarin), fluorescent microsphere, luminescent nanocrystal, Marina blue™(Life technologies), Cascade blue™(Life technologies), Cascade yellow™(Life technologies), Pacific blue™(Life technologies), Oregon green™(Life technologies), Tetramethylrhodamine, Rhodamine, Texas red™(Life technologies), rare earth element chelates, or any combination or derivatives thereof.

Other representative acceptor molecules can include, but are not limited to sgGFP, sgBFP, BFP blue shifted GFP (Y66H), Cyan GFP, DsRed, monomeric RFP, EBFP, ECFP, GFP (S65T), GFP red shifted (rsGFP), non-UV excitation (wtGFP), UV excitation (wtGFP), GFPuv, HcRed, rsGFP, Sapphire GFP, sgBFP™, sgBFP™ (super glow BFP), sgGFP™, sgGFP™ (super glow GFP), Yellow GFP, semiconductor nanoparticles (*e.g.*, raman nanoparticles), 1,5 IAEDANS; 1,8-ANS; 4-Methylumbelliferone; 5-carboxy-2,7-dichlorofluorescein; 5-Carboxyfluorescein (5-FAM); 5-Carboxynapthofluorescein; 5-Carboxytetramethylrhodamine (5-TAMRA); 5-FAM (5-Carboxyfluorescein); 5-HAT (Hydroxy Tryptamine); 5-Hydroxy Tryptamine (HAT); 5-ROX (carboxy-X-rhodamine); 5-TAMRA (5-Carboxytetramethylrhodamine); 6-Carboxyrhodamine 6G; 6-CR 6G; 6-JOE; 7-Amino-4-methylcoumarin; 7-Aminoactinomycin D (7-AAD); 7-Hydroxy-4-methylcoumarin; 9-Amino-6-chloro-2-methoxyacridine; ABQ; Acid Fuchsin; ACMA (9-Amino-6-chloro-2-methoxyacridine); Acridine Orange; Acridine Red; Acridine Yellow; Acriflavin; Acriflavin Feulgen SITSA; Aequorin (Photoprotein); AFPs (AutoFluorescent Protein; Quantum Biotechnologies); Alexa Fluor 350™; Alexa Fluor 430™; Alexa Fluor 488™; Alexa Fluor 532™; Alexa Fluor 546™; Alexa Fluor 568™; Alexa Fluor 594™; Alexa Fluor 633™; Alexa Fluor 647™; Alexa Fluor 660™; Alexa Fluor 680™; Alizarin Complexon; Alizarin Red; Allophycocyanin (APC); AMC, AMCA-S; AMCA (Aminomethylcoumarin); AMCA-X; Aminoactinomycin D; Aminocoumarin; Aminomethylcoumarin (AMCA); Anilin Blue; Anthrocyl stearate; APC (Allophycocyanin); APC-Cy7; APTRA-BTC; APTS; Astrazon Brilliant Red 4G; Astrazon Orange R; Astrazon Red 6B; Astrazon Yellow 7 GLL; Atabrine; ATTO-TAG™ CBQCA; ATTO-TAG™ FQ; Auramine; Aurophosphine G; Aurophosphine; BAO 9 (Bisaminophenyloxadiazole); BCECF (high pH); BCECF (low pH); Berberine Sulphate; Beta Lactamase; Bimane; Bisbenzamide; Bisbenzimide (Hoechst); bis-BTC; Blancophor FFG; BlancophorSV; BOBO™-1; BOBO™-3; Bodipy 492/515; Bodipy 493/503; Bodipy 500/510; Bodipy 505/515; Bodipy 530/550; Bodipy 542/563; Bodipy 558/568; Bodipy 564/570; Bodipy 576/589; Bodipy 581/591; Bodipy 630/650-X; Bodipy 650/665-X; Bodipy 665/676; Bodipy Fl; Bodipy FL ATP; Bodipy Fl-Ceramide; Bodipy R6G SE; Bodipy TMR; Bodipy TMR-X conjugate; Bodipy TMR-X, SE; Bodipy TR; Bodipy TR ATP; Bodipy TR-X SE; BO-PRO™-1; BO-PRO™-3; Brilliant Sulphoflavin FF; BTC; BTC-5N; Calcein; Calcein Blue; Calcium Crimson™; Calcium Green; Calcium Green-1 $Ca^{2+}$ Dye; Calcium Green-2 $Ca^{2+}$; Calcium Green-5N $Ca^{2+}$; Calcium Green-C18 $Ca^{2+}$; Calcium Orange; Calcofluor White; Carboxy-X-rhodamine (5-ROX); Cascade Blue™; Cascade Yellow; Catecholamine; CCF2 (GeneBlazer); CFDA; Chlorophyll; Chromomycin A; Chromomycin A; CL-NERF; CMFDA; Coumarin Phalloidin; C-phycocyanine; CPM Methylcoumarin; CTC; CTC Formazan; Cy2™; Cy3.18; Cy3.5™; Cy3™; Cy5.18; Cy5.5™; Cy5™; Cy7™; cyclic AMP Fluorosensor (FiCRhR); Dabcyl; Dansyl; Dansyl Amine; Dansyl Cadaverine; Dansyl Chloride; Dansyl DHPE; Dansyl fluoride; DAPI; Dapoxyl; Dapoxyl 2; Dapoxyl 3' DCFDA; DCFH (Dichlorodihydrofluorescein Diacetate); DDAO; DHR (Dihydorhodamine 123); Di-4-ANEPPS; Di-8-ANEPPS (non-ratio); DiA (4-Di-16-ASP); Dichlorodihydrofluorescein Diacetate (DCFH); DiD-Lipophilic Tracer; DiD (DilC18(5)); DIDS; Dihydrorhodamine 123 (DHR); Dil (DilC18(3)); Dinitrophenol; DiO (DiOC18(3)); DiR; DiR (DilC18(7)); DM-NERF (high pH); DNP; Dopamine; DTAF; DY-630-NHS; DY-635-NHS; ELF 97; Eosin; Erythrosin; Erythrosin ITC; Ethidium Bromide; Ethidium homodimer-1 (EthD-1); Euchrysin; EukoLight; Europium (III) chloride; EYFP; Fast Blue; FDA; Feulgen (Pararosaniline); FIF (Formaldehyd Induced Fluorescence); FITC; Flazo Orange; Fluo-3; Fluo-4; Fluorescein (FITC); Fluorescein Diacetate; Fluoro-Emerald; Fluoro-Gold (Hydroxystilbamidine); Fluor-Ruby; FluorX; FM 1-43™; FM 4-46; Fura Red™ (high pH); Fura Red™/Fluo-3; Fura-2; Fura-2/BCECF; Genacryl Brilliant Red B; Genacryl Brilliant Yellow 10GF; Genacryl Pink 3G; Genacryl Yellow 5GF; GeneBlazer (CCF2); Gloxalic Acid; Granular blue; Haematoporphyrin; Hoechst 33258; Hoechst 33342; Hoechst 34580; HPTS; Hydroxycoumarin; Hydroxystilbamidine (FluoroGold); Hydroxytryptamine; Indo-1, high calcium; Indo-1, low calcium; Indodicarbocyanine (DiD); Indotricarbocyanine (DiR); Intrawhite Cf; JC-1; JO-JO-1; JO-PRO-1; LaserPro; Laurodan; LDS 751 (DNA); LDS 751 (RNA); Leucophor PAF; Leucophor SF; Leucophor WS; Lissamine Rhodamine; Lissamine Rhodamine B; Calcein/Ethidium homodimer; LOLO-1; LO-PRO-1; Lucifer Yellow; Lyso Tracker Blue; Lyso Tracker Blue-White; Lyso Tracker Green; Lyso Tracker Red; Lyso Tracker Yellow; LysoSensor Blue; LysoSensor Green; LysoSensor Yellow/Blue; Mag Green; Magdala Red (Phloxin B); Mag-Fura Red; Mag-Fura-2; Mag-Fura-5; Mag-Indo-1; Magnesium Green; Magnesium Orange; Malachite Green; Marina Blue; Maxilon Brilliant Flavin 10 GFF; Maxilon Brilliant Flavin 8 GFF; Merocyanin; Methoxycoumarin; Mitotracker Green FM; Mitotracker Orange; Mitotracker Red; Mitramycin; Monobromobimane; Monobromobimane (mBBr-GSH); Monochlorobimane; MPS (Methyl Green Pyronine Stilbene); NBD; NBD Amine; Nile Red; Nitrobenzoxadidole; Noradrenaline; Nuclear Fast Red; Nuclear Yellow; Nylosan Brilliant lavin E8G; Oregon Green; Oregon Green 488-X; Oregon Green™; Oregon Green™ 488; Oregon Green™ 500; Oregon Green™ 514; Pacific Blue; Pararosaniline (Feulgen); PBFI; PE-Cy5; PE-Cy7; PerCP; PerCP-Cy5.5; PE-TexasRed [Red 613]; Phloxin B (Magdala Red); Phorwite AR; Phorwite BKL; Phorwite Rev; Phorwite RPA; Phosphine 3R; PhotoResist; Phycoerythrin B [PE]; Phycoerythrin R [PE]; PKH26 (Sigma); PKH67; PMIA; Pontochrome Blue Black; POPO-1; POPO-3; PO-PRO-1; PO-PRO-3; Primuline; Procion Yellow; Propidium Iodid (PI); PyMPO; Pyrene; Pyronine; Pyronine B; Pyrozal Brilliant Flavin 7GF; QSY 7; Quinacrine Mustard; Red 613 [PE-TexasRed]; Resorufin; RH 414; Rhod-2; Rhodamine; Rhodamine 110; Rhodamine 123; Rhodamine 5 GLD; Rhodamine 6G; Rhodamine B; Rhodamine B 200; Rhodamine B extra; Rhodamine BB; Rhodamine BG; Rhodamine Green; Rhodamine Phallicidine; Rhodamine Phalloidine; Rhodamine Red; Rhodamine WT; Rose Bengal; R-phycocyanine; R-phycoerythrin (PE); S65A; S65C; S65L; S65T; SBFI; Serotonin; Sevron Brilliant Red 2B; Sevron Brilliant Red 4G; Sevron Brilliant Red B; Sevron Orange; Sevron Yellow L; SITS; SITS (Primuline); SITS (Stilbene Isothiosulphonic Acid); SNAFL calcein; SNAFL-1; SNAFL-2; SNARF calcein; SNARF1; Sodium Green; SpectrumAqua; SpectrumGreen; SpectrumOrange; Spectrum Red; SPQ (6-methoxy-N-(3-sulfopropyl)quinolinium); Stilbene; Sulphorhodamine B can C; Sulphorhodamine Extra; SYTO 11; SYTO 12; SYTO 13; SYTO 14; SYTO 15; SYTO 16; SYTO 17; SYTO 18; SYTO 20; SYTO 21; SYTO 22; SYTO 23; SYTO 24; SYTO 25; SYTO 40; SYTO 41; SYTO 42; SYTO 43; SYTO 44; SYTO 45; SYTO 59; SYTO 60; SYTO 61; SYTO 62; SYTO 63; SYTO 64; SYTO 80; SYTO 81; SYTO 82; SYTO 83; SYTO 84; SYTO 85; SYTOX Blue; SYTOX Green; SYTOX Orange; Tetracycline; Tetramethylrhodamine (TRITC); Texas Red™; Texas Red-X™ conjugate; Thiadicarbocyanine (DiSC3); Thiazine Red R; Thiazole Orange; Thioflavin 5; Thioflavin S; Thioflavin TCN; Thiolyte; Thiozole Orange; Tinopol CBS (Calcofluor White); TMR; TO-PRO-1; TO-PRO-3; TO-PRO-5; TOTO-1; TOTO-3; TriColor (PE-Cy5); TRITC TetramethylRodamineIsoThioCyanate; True Blue; TruRed; Ultralite; Uranine B; Uvitex SFC; WW 781; X-Rhodamine; XRITC; Xylene Orange; Y66F; Y66H; Y66W; YO-PRO-1; YO-PRO-3; YOYO-1 ; YOYO-3 ,Sybr Green, Thiazole orange (interchelating dyes), or combinations thereof.

Alternatively, the acceptor molecule may be a fluorescent nanocrystal. Nanocrystals, have several advantages over organic molecules as fluorescent labels, including resistance to photodegradation, improved brightness, non- toxicity, and size dependent, narrow emission spectra that enables the monitoring of several processes simultaneously. Additionally, the absorption spectrum of nanocrystals is continuous above the first peak, enabling all sizes, and hence all colors, to be excited with a single excitation wavelength.

Fluorescent nanocrystals may be attached, or "bioconjugated", to proteins in a variety of ways. For example, the surface cap of a "quantum dot" may be negatively charged with carboxylate groups from either dihydrolipoic acid (DHLA) or an amphiphilic polymer. Proteins can be conjugated to the DHLA-nanocrystals electrostatically, either directly or via a bridge consisting of a positively charged leucine zipper peptide fused to recombinant protein. The latter binds to a primary antibody with specificity for the intended target. Alternatively, antibodies, streptavidin, or other proteins are coupled covalently to the polyacrylate cap of the nanocrystal with conventional carbodiimide chemistry.

There are colloidal methods to produce nanocrystals, including cadmium selenide, cadmium sulfide, indium arsenide, and indium phosphide. These quantum dots can contain as few as 100 to 100,000 atoms within the quantum dot volume, with a diameter of 10 to 50 atoms. Some quantum dots are small regions of one material buried in another with a larger band gap. These can be so-called core-shell structures, for example, with CdSe in the core and ZnS in the shell or from special forms of silica called ormosil. Conversely, smaller dots emit bluer (higher energy) light. The coloration is directly related to the energy levels of the quantum dot. Quantitatively speaking, the bandgap energy that determines the energy (and hence color) of the fluoresced light is inversely proportional to the square of the size of the quantum dot. Larger quantum dots have more energy levels which are more closely spaced. This allows the quantum dot to absorb photons containing less energy, i.e. those closer to the red end of the spectrum.

The acceptor molecule may also be a fluorescent microsphere. These are typically made from polymers, and contain fluorescent molecules (for example fluorescein GFP or YFP) incorporated into the polymer matrix, which can be conjugated to a variety of reagents. Fluorescent microspheres may be labeled internally or on the surface. Internal labeling produces very bright and stable particles with typically narrow fluorescent emission spectra. With internal labeling, surface groups remain available for conjugating ligands (for example, proteins) to the surface of the bead. Internally-labeled beads are used extensively in imaging applications, as they display a greater resistance to photobleaching.

Carboxylate-modified fluorescent microspheres are suitable for covalent coupling of proteins using water-soluble carbodiimide reagents such as 1-ethyl-3-(3-dimethylaminopropyl)carbodiimide hydrochloride (EDAC). Sulfate fluorescent microspheres are relatively hydrophobic and will passively and nearly irreversibly adsorb almost any protein. Aldehyde-sulfate fluorescent microspheres are sulfate microspheres that have been modified to add surface aldehyde groups, and react with proteins.

Finally, the acceptor molecule may be a luminescent microsphere. These are typically made from polymers, which contain luminescent molecules (for example complexes of europium or platinum) incorporated into the polymer matrix, which can be conjugated to a variety of reagents.

Criteria which should be considered in determining suitable pairings for BRET is the relative emission/fluorescence spectrum of the acceptor molecule compared to that of the bioluminescent donor molecule. The emission spectrum of the bioluminescent protein should overlap with the absorbance spectrum of the acceptor molecule such that the light energy from the bioluminescent protein luminescence emission is at a wavelength that is able to excite the acceptor molecule and thereby promote acceptor molecule fluorescence when the two molecules are in a proper proximity and orientation with respect to one another. For example, it has been demonstrated that an Renilla luciferase/EGFP pairing is not as good as an Renilla luciferase/EYEF pairing based on observable emission spectral peaks.

The bioluminescent protein emission can be manipulated by modifications to the substrate. In the case of luciferases the substrate is coelenterazine. The rationale behind altering the bioluminescent protein emission is to improve the resolution between donor emission and acceptor emission. The original BRET system uses the *Renilla* luciferase as donor, EYFP (or Topaz) as the acceptor and coelenterazine derivative as the substrate. These components when combined in a BRET assay generate maximal light in the 475-485 nm range for the bioluminescent protein and the 525-535 nm range for the acceptor molecule, giving a spectral resolution of 40-60 nm.

*Renilla* luciferase generates a broad emission peak overlapping substantially the GFP emission, which in turn contributes to decrease the signal to noise of the system. Various coelenterazine derivatives are known in the art, including coel400a, that generate light at various wavelengths (distinct from that generated by the wild type coelenterazine) as a result of *Renilla* luciferase activity. A skilled person in the art would appreciate that because the light emission peak of the donor has changed, it is necessary to select an acceptor molecule which will absorb light at this wavelength and thereby permit efficient energy transfer. Spectral overlapping between light emission of the donor and the light absorption peak of the acceptor is one condition among others for an efficient energy transfer. Class 3 and 1 GFPs are known to absorb light at 400 nm and re-emit between 505-511 nm. This results in a wavelength difference between donor and acceptor emissions of approximately 111 nm.

Bioluminescent donor and acceptor molecule pairs are well known in the art and are well within the reach of a person of skill in art. Based on the knowledge regarding the ability of donor to generate luminescence and the ability of an acceptor to accept energy emitted as a result of the activity of a bioluminescent donor protein, and re-emit it as light energy or based on transfer of excited-state energy between such pairs of molecules, spectral overlap, the relative orientation, and the distance between the donor and acceptor many bioluminescent proteins and acceptor molecule pairs can be selected by a person of skill in art.

The present invention also relates to an expression vector comprising an acid nucleic encoding a voltage-dependent ion channel fusion subunit as described above. The nucleic acid or polynucleotide of the present invention may be inserted into several commercially available expression vectors. Non-limiting examples include prokaryotic plasmid vectors, such as the pUC-series, pBluescript (Stratagene), the pET-series of expression vectors (Novagen) or pCRTOPO (Invitrogen) and vectors compatible with an expression in mammalian cells like pREP (Invitrogen), pcDNA3 (Invitrogen), pCEP4 (Invitrogen), pMC1neo (Stratagene), pXT1 (Stratagene), pSG5 (Stratagene), EBO-pSV2neo, pBPV-1, pdBPVMMTneo, pRSVgpt, pRSVneo, pSV2-dhfr, pIZD35, pLXIN, pSIR (Clontech), pIRES-EGFP (Clontech), pEAK-10 (Edge Biosystems) pTriEx-Hygro (Novagen) and pCINeo (Promega). Examples for plasmid vectors suitable for *Pichia pastoris* comprise e.g. the plasmids pAO815, pPIC9K and pPIC3.5K (all Intvitrogen). The nucleic acid or polynucleotide of the present invention referred to above may also be inserted into vectors such that a translational fusion with another polynucleotide is generated. The other polynucleotide may encode a protein which may e.g. increase the solubility and/or facilitate the purification of the fusion protein. Non-limiting examples include pET32, pET41, pET43. The vectors may also contain an additional expressible polynucleotide coding for one or more chaperones to facilitate correct protein folding. Suitable bacterial expression hosts comprise e.g. strains derived from BL21 (such as BL21(DE3), BL21(DE3)PlysS, BL21(DE3)RIL, BL21(DE3)PRARE) or Rosetta®. In preferred embodiments, expression vector according to the invention is a DNA or RNA vector, capable of transforming eukaryotic host cells and effecting stable or transient expression of said TRP channel fusion subunit, and wherein said vector is a plasmid, a virus like adenovirus, adeno associated virus (AVV), lentiviral, Epstein-Barr , Herpes Simplex, Papilloma, Polyoma, Retro, SV40, Vaccinia, any retroviral vector, influenza viral vector and other non-viral vectors including naked DNA, or liposomes.

Generally, vectors can contain one or more origin of replication (ori) and inheritance systems for cloning or expression, one or more markers for selection in the host, e.g., antibiotic resistance, and one or more expression cassettes. Suitable origins of replication (ori) include, for example, the Col E1, the SV40 viral and the M 13 origins of replication. The coding sequences inserted in the vector can e.g. be synthesized by standard methods, or isolated from natural sources. Ligation of the coding sequences to transcriptional regulatory elements and/or to other amino acid encoding sequences can be carried out using established methods. Transcriptional regulatory elements (parts of an expression cassette) ensuring expression in prokaryotes or eukaryotic cells are well known to those skilled in the art.

These elements comprise regulatory sequences ensuring the initiation of the transcription (e.g., translation initiation codon, promoters, enhancers, and/or insulators), internal ribosomal entry sites (IRES) and optionally poly-A signals ensuring termination of transcription and stabilization of the transcript. Additional regulatory elements may include transcriptional as well as translational enhancers, and/or naturally-associated or heterologous promoter regions. Preferably, the polynucleotide of the invention is operatively linked to such expression control sequences allowing expression in prokaryotes or eukaryotic cells. The vector may further comprise nucleotide sequences encoding secretion signals as further regulatory elements. Such sequences are well known to the person skilled in the art. Furthermore, depending on the expression system used, leader sequences capable of directing the expressed polypeptide to a cellular compartment may be added to the coding sequence of the polynucleotide of the invention. Such leader sequences are well known in the art.

Possible examples for regulatory elements ensuring the initiation of transcription comprise the cytomegalovirus (CMV) promoter, SV40-promoter, RSV-promoter (Rous sarcome virus), the lacZ promoter, human elongation factor 1α-promoter, CMV enhancer, CaM-kinase promoter, the *Autographa californica* multiple nuclear polyhedrosis virus (AcMNPV) polyhedral promoter or the SV40-enhancer. For the expression in prokaryotes, a multitude of promoters including, for example, the tac-lac-promoter, the lacUV5 or the trp promoter, has been described. Examples for further regulatory elements in prokaryotes and eukaryotic cells comprise transcription termination signals, such as SV40-poly-A site or the tk-poly-A site or the SV40, lacZ and AcMNPV polyhedral polyadenylation signals, downstream of the polynucleotide.

According to the present invention, nucleotide sequence encoding the channel fusion subunit may be operably linked to a promoter and optionally to an enhancer. Preferred promoters may be selected from but are not limited to promoter is CMV promoter, RSV promoter, SV40 promoter, adenovirus promoter, adenovirus E1A, Heat Shock protein promoter, a promoter from *Mycobacteria* genes and RNA, *Mycobacterium bovis* MPB70, MPB59, or MPB64 antigen promoter, PI promoter from bacteriophage Lambda, a tac promoter, a trp promoter, a lac promoter, a lacUV5 promoter, an Ipp promoter, a $P_L\lambda$ promoter, a $P_R\lambda$ promoter, a rac5 promoter, a β-lactamase, a recA promoter, a SP6 promoter, a T7 promoter, a, a metallothionine promoter, a growth hormone promoter, a hybrid promoter between a eukaryotic promoter and a prokaryotic promoter, ubiquitin promoter, E2F, CEA, MUC1/DF3, α-fetoprotein, erb-B2, surfactant, tyrosinase, PSA, TK, p21, hTERT, hKLK2, probasin or a cyclin gene derived promoter and wherein said enhancer is selected from immediate early enhancer, β-actin, or an adenovirus inverted terminal repeats (ITR). Enhancers may be selected from immediate early enhancer, b-actin, Adenovirus inverted terminal repeats (ITR) and the like.

Furthermore, it is preferred that the vector of the invention comprises a selectable marker. Examples of selectable markers include neomycin, ampicillin, and hygromycine, kanamycine resistance and the like. Specifically-designed vectors allow the shuttling of DNA between different hosts, such as bacteria-fungal cells or bacteria-animal cells (e.g. the Gateway® system available at Invitrogen).

According to the present invention, the subunit of a voltage-dependent cation channel or voltage-dependent anion channel comprises 2 to 24 transmembrane domains. The bioluminescent protein can form part of the C-terminus or the N-terminus of the nucleic acid of the present invention. The acceptor molecule also can form part of the C-terminus or the N-terminus of the nucleic acid of the present invention. The acceptor molecule cannot be in the same region as the donor molecule when part of the same nucleic acid construct, however, the acceptor molecule can be in the equivalent region as the donor molecule. For example, the donor can form part of the C-terminus whilst the acceptor molecule can form part of the N-terminus and vice versa.

A person of skill in the art can readily determine the N-terminal end, C-terminal end, transmembrane domains, non-transmembrane loops (domains) and intracellular domains within the test nucleic acid. For example, a variety of bioinformatics approaches may be used to determine the location and topology of transmembrane domains, non-transmembrane domains and intracellular domains in a nucleic acid or an amino acid, based on its sequence and similarity with known domain of voltage dependent receptor sequences. Alignments and nucleic acid and/or amino acid sequence comparisons are routinely performed in the art, for example, by using the BLAST program or the clustalw programs. Based on alignments with known transmembrane domain or intracellular domain-containing nucleic acids and/or proteins, it is possible for one skilled in the art to predict the location of such domains. Furthermore, the 3 dimensional structures of many such receptors are known and catalogued in public information sources. Based on analysis and comparisons with such 3D structures, it may be possible to predict the location and topology of transmembrane domains in other such nucleic acid sequences encoding subunits of voltage dependent receptors. There are also many programs available for predicting the location and topology of transmembrane domains in proteins. For example, one may use one or a combination of the TMpred which predicts membrane spanning segments: TopPred which predicts the topology of membrane proteins; PRFDATOR, which predicts secondary structure from single and multiple sequences; TMAP, which predicts transmembrane regions of proteins from multiply aligned sequences; and numerous other programs which predicts transmembrane regions from single sequences.

The present invention also relates to a cell, genetically engineered with the nucleic acid or polynucleotide of the present invention or the vector of the present invention. The cell may be in cell culture or part of a host. Said cell or said host may be produced by introducing said polynucleotide or vector(s) into a cell or host which upon its/their presence mediates the expression of the polypeptide (*i.e.,* fusion subunit) encoded by said nucleic acid or polynucleotide. The cell or host may be any prokaryote or eukaryotic cell. The host may be any prokaryote or eukaryotic cell. Suitable eukaryotic host may be a mammalian cell, an amphibian cell, a fish cell, an insect cell, a fungal cell or a plant cell. Eukaryotic cell may be an insect cell such as a *Spodoptera frugiperda* cell, a yeast cell such as a *Saccharomyces cerevisiae* or *Pichia pastoris* cell, a fungal cell such as an *Aspergillus* cell or a vertebrate cell. Suitable prokaryotes may be *E. coli* (e.g., *E coli* strains HB101, DH5a, XL1 Blue, Y1090 and JM101), *Salmonella typhimurium, Serratia marcescens, Burkholderia glumae, Pseudomonas putida, Pseudomonas fluorescens, Pseudomonas stutzeri, Streptomyces lividans, Lactococcus lactis, Mycobacterium smegmatis* or *Bacillus subtilis.*

The present invention is thus also directed to a recombinant host cell containing an expression vector for expression of voltage-dependent ion channel subunit, wherein said vector contains a polynucleotide comprising a nucleic acid sequence encoding the voltage-dependent ion channel subunit or a functionally equivalent active fragment thereof as described above. The expressed voltage-dependent ion channel subunit can be in the form of a fusion protein, for example a fusion to a bioluminescent donor molecule, and/or bioluminescent acceptor molecule. Methods of producing polynucleotides and vectors (e.g., viral and non-viral) are well known in the art. Preferably, the said vector is a plasmid, cosmid, virus, bacteriophage or another vector used conventionally in genetic engineering.

Recombinant host cells according to the present invention thus comprise an expression vector wherein said channel fusion subunit is expressed and is able to co-assemble with other homomeric or heteromeric channel subunits *in vitro* and *in vivo* to form a functional channel. The present invention embodies a process for the production of said channel fusion subunits as described above comprising culturing said recombinant cell according to the invention, and expressing said channel fusion subunit.

Recombinant host cells according to the present invention preferably present as a stable line.

The present invention also provides a fusion subunit encoded by the nucleic acid according to the present invention, wherein said N-terminal extremity or C-terminal extremity is bound to at least one bioluminescent donor protein and/or at least one acceptor protein. It is also within the skill of a person of ordinary skill in art, to incorporate the donor and acceptor on parts of two different subunits, instead of on a single subunit.

The current invention further relates to a cell free and as live cell assay which is more sensitive and existing chemical detection systems such as fluorescence and a gas chromatographic technique with flame ionization detection (GC-FID). The present invention relates to methods and nucleic acids for detecting a compound in a sample. In particular, the present invention relates to the use of a cell-free or whole cell composition comprising at least one voltage-dependent ion channel subunit in combination or coupled or bound to a nucleotide sequence encoding at least one bioluminescent donor molecule and/or bound to a nucleotide sequence encoding at least one fluorescent acceptor molecule.

According to the present invention, the cell free composition comprises the voltage-dependent ion channel fusion subunit as described above, wherein said fusion subunit is embedded in a lipid bilayer, preferably in a bilayer of a liposome. Such lipid bilayer comprises two layers of, typically amphiphilic, with a common hydrophobic bilayer interior and two hydrophilic surfaces. The lipid bilayer can be naturally occurring or artificial. Such lipid bilayer may be a cellular or bio-membrane for example from mammalian or yeast cell membrane, into which voltage-dependent ion channel fusion subunit is inserted.

Methods for preparing cell-free compositions from cells are well-known in the art and consist in obtaining recombinant cells as described above and disrupting the membrane of the cells. These methods generally include repeated cycles of freezing and thawing, grinding, treatment of cells with ultrasound in a sonicator device, homogenization, and use of a French press, the addition of detergent and/or enzymes, glass-bead lysis, differential centrifugation, and several density gradient procedures using a variety of gradient media. These techniques are *inter alia* described in details in "Current Protocols in Protein Science"; John E. Caligan; Ben M. Dunn; Hidde L. Ploegh; David W. Speicher; Paul T. Wingfield; Wiley and Sons). For isolating or preparing cell membrane extracts, a combination of these methods is usually employed. Generally, cells are lysed either by mechanical means, or using detergents, and the membrane fractions isolated via differential centrifugation. Liposomes containing recombinant cell as per the invention may be created by disrupting the phospholipid membrane of cells expressing the protein in water, for example by sonication. The phospholipids would reassemble into liposomal spheres containing a core of aqueous solution. Low shear rates would create multilamellar liposomes, which have many layers. Continued high-shear sonication would form smaller unilamellar liposomes, more suited to the application of the present invention.

Assay for BRET

In a preferred embodiment, the energy transfer occurring between the bioluminescent protein and acceptor molecule is presented as calculated ratios from the emissions measured using optical filters (one for the acceptor molecule emission and the other for the bioluminescent protein emission) that select specific wavelengths (see equation 1).
Equation 1:

$$Ea/Ed = BRET\ ratio\ (1)$$

where Ea is defined as the acceptor molecule emission intensity (emission light is selected using a specific filter adapted for the emission of the acceptor) and Ed is defined as the bioluminescent protein emission intensity (emission light is selected using a specific filter adapted for the emission of the bioluminescent protein).

It should be readily appreciated by those skilled in the art that the optical filters may be any type of filter that permits wavelength discrimination suitable for BRET. For example, optical filters used in accordance with the present invention can be interference filters, long pass filters, short pass filters, etc. Intensities (usually in counts per second (CPS) or relative luminescence units (RLU)) of the wavelengths passing through filters can be quantified using either a photo-multiplier tube (PMT) or a CCD camera. The quantified signals are subsequently used to calculate BRET ratios and represent energy transfer efficiency. The BRET ratio increases with increasing intensity of the acceptor emission.

Generally, a ratio of the acceptor emission intensity over the donor emission intensity is determined (see equation 1), which is a number expressed in arbitrary units that reflects energy transfer efficiency. The ratio increases with an increase of energy transfer efficiency.

Energy transfer efficiencies can also be represented using the inverse ratio of donor emission intensity over acceptor emission intensity (see equation 2). In this case, ratios decrease with increasing energy transfer efficiency. Prior to performing this calculation the emission intensities are corrected for the presence of background light and auto-luminescence of the substrate. This correction is generally made by subtracting the emission intensity, measured at the appropriate wavelength, from a control sample containing the substrate but no bioluminescent protein, acceptor molecule or polypeptide of the invention.
Equation 2:

$$Ed/Ea = BRET\ ratio\ (2)$$

where Ea and Ed are as defined above.

The light intensity of the bioluminescent protein and acceptor molecule emission can also be quantified using a monochromator-based instrument such as a spectrofluorometer, a charged coupled device (CCD) camera or a diode array detector. Using a spectrofluorometer, the emission scan is performed such that both bioluminescent protein and acceptor molecule emission peaks are detected upon addition of the substrate. The areas under the peaks represent the relative light intensities and are used to calculate the ratios, as outlined above. Any instrument capable of measuring lights for the bioluminescent protein and acceptor molecule from the same sample can be used to monitor the BRET system of the present invention.

In an alternative embodiment, the energy transfer efficiency is represented using a ratiometric measurement which only requires one optical filter for the measurement. In this case, light intensity for the donor or the acceptor is determined using the appropriate optical filter and another measurement of the samples is made without the use of any filter (intensity of the open spectrum). In this latter measurement, total light output (for all wavelengths) is quantified. Ratio calculations are then made using either equation 3 or 4. For the equation 3, only the optical filter for the acceptor is required. For the equation 4, only the optical filter for the donor is required.
Equation 3:

$$Ea/Eo\text{-}Ea = BRET\ ratio\ or = Eo\text{-}Ea/Ea\ (3)$$

Equation 4:

$$Eo\text{-}Ed/Ed = BRET\ ratio\ or = Ed/Eo\text{-}Ed\ (4)$$

where Ea and Ed are as defined above and Eo is defined as the emission intensity for all wavelengths combined (open spectrum).

It should be readily apparent to one skilled in the art that further equations can be derived from equations 1 through 4. For example, one such derivative involves correcting for background light present at the emission wavelength for bioluminescent protein and/or acceptor molecule.

In performing a BRET assay, light emissions can be determined from each well using any appropriate instrument known by one of ordinary skill in the art. BRET measurement can be performed for instance using readers that can detect at least the acceptor molecule emission and preferably two wavelengths (for the acceptor molecule and the bioluminescent protein) or more.

In an embodiment of the invention, BRET is detected using a microfluidics device. Microfluidics devices conveniently require only an aliquot of the sample, generally not more than about 50 μL, to be transferred to the sample reservoir of the micro fluidics device. This is performed either manually or by pneumatic injection via a syringe, capillary or the like.

An automated luminescence biochip device using microfluidics may be used to perform all the necessary BRET reaction steps. Automating BRET reactions in a microfluidic biochip platform is desirable as this avoids multiple manual handling steps and reduces human time and effort in performing experiments. The microfluidics device may contain a self-contained disposable biochip with patterned microchannels and compartments having storage means for storing a plurality of samples, reagents, and substrates. The steps of transferring sequentially at least one of the samples, or reagents, and then luminescent substrate from compartments through microchannels to the reaction sites could be automated. The luminescent substrates would then react with the donor molecules resulting in luminescence, which would be detected by an optical detector. An example of a microfluidics device for detecting luminescence is described in US 6,949,377.

The present invention further relates to a process of screening in real time agonist or antagonist of the voltage-dependent ion channel.

Recombinant DNA techniques are used to isolate a nucleic acid encoding a voltage-dependent ion channel subunit, for example, any of the channels listed in Tables 1-11. Standard recombinant DNA protocols are used to clone the nucleic acids encoding the nucleic acids into any plasmid that facilitates the expression of an in frame fusion of the nucleic acid bound to a nucleotide sequence encoding at least one bioluminescent or fluorescent donor molecule and/or bound to a nucleotide sequence encoding at least one fluorescent acceptor molecule. Any means now known or discovered in the future are used to co-introduce the expression constructs into a host cell. The host cells are propagated using known culturing methods, and fusion proteins are co-expressed in the recombinant host cells.

To detect agonist activity, samples of the transfected cells can be added to a 96-well plate (Costar 3912). Various concentrations of known agonists or test compounds are added to the wells with the cell samples. Following a short incubation period, for example about 20 minutes, the BRET reaction is started by submitting cell sample to a stimulus appropriate for triggering the BRET. The stimulus may be selected for instance from the addition of a substrate, an increase of temperature, and an irradiation with radiofrequences. One of ordinary skill in the art is able to adapt the stimulus to the voltage-dependent ion channel, the donor molecule and/or the fluorescent acceptor molecule. The emission of light at various wavelengths is measured on a Mithras LB940 plate reader (Berthold, Germany) or instrument with similar capabilities. For example, BRET optimized filters (Berthold, Germany) are used to detect luciferase emission at 395 nm and fluorescent emission at 510 nm wavelength. The emissions are measured for one second each. Data from the BRET assays are fit to the equation $R=D+(A-D)/(1+(x/c))$, where A=minimum response, D=maximum response and c=EC50 (R=response, x=concentration of ligand) using the Prism 4.0 software (GraphPad Software, Dan Diego, Calif., USA). The readings from three wells are used to generate each data point. The data points for cells treated with test compounds are compared to the data points for cells not treated with known agonists or antagonists. A comparison of the BRET signals to untreated cells, or cells treated with known agonists or antagonists, is used to characterize the test compound as a ligand, as well as if the ligand is an agonist, antagonist, or inverse agonist of the voltage-dependent ion channel subunit.

To detect antagonist activity, samples of the transfected cells are added to a 96 well plate. A known agonist is added to the cells, and the cells are incubated for a period of time, such as for 10 minutes. Following incubation with the known agonist, various concentrations of a known antagonist or test compound are added to the cell samples. Cells are incubated with the known antagonist or test compound, for example for 10 minutes. Following a short incubation period, for example about 20 minutes, the BRET reaction is started by submitting the cell sample to a stimulus appropriate for triggering the BRET. The stimulus may be selected for instance from the addition of a substrate, an increase of temperature, and an irradiation with radiofrequences. One of ordinary skill in the art is able to adapt the stimulus to the voltage-dependent ion channel, the donor molecule and/or the fluorescent acceptor molecule. The emission of light at various wavelengths is measured on a Mithras LB940 plate reader (Berthold, Germany) or instrument with similar capabilities. For example, BRET optimized filters (Berthold, Germany) are used to detect luciferase emission at 395 nm and fluorescent emission at 510 nm wavelength. The emissions are measured for one second each. Data from the BRT BRET assays are fit to the equation $R=D+(A-D)/(1+(x/c))$, where A=minimum response, D=maximum response and c=EC50 (R=response, x=concentration of ligand) using the Prism 4.0 software (GraphPad Software, Dan Diego, Calif., USA). The readings from three wells are used to generate each data point. The data points for cells treated with test compounds are compared to the data points for cells not treated with known agonists. A comparison of the BRET signals to untreated cells, or cells treated with known agonists, is used to characterize the test compound as a ligand, as well as if the ligand is an antagonist of the voltage-dependent ion channel subunit or more specifically TRP channel.

The current invention provides a method of screening in real time a compound candidate capable of activating or inhibiting channel, comprising: (i) contacting the candidate with the recombinant host cell according to the invention, or a cell free composition according to the invention, (ii) providing a substrate of the bioluminescent donor molecule; (iii) measuring the variation of BRET signal.

In a further aspect, the present invention provides a kit for screening agonist or inhibitor compound candidate of voltage-dependent ion channel comprising a nucleic acid or a polynucleotide of the invention, a vector of the invention, a recombinant cell or a host cell of the invention, or a cell-free composition or a composition of the invention, and/or a biosensor of the invention.

The present invention can be used to detect a wide variety of compounds which may act as agonists or antagonists to the voltage-dependent ion channels. To that effect the present invention also provides a biosensor or a device for the detection of an analyte that combines a biological component with a physicochemical detector component. It typically consists of three parts, firstly at least one nucleotide subunit encoding a voltage-dependent ion channel subunit. Second, a transducer or detector element, which works in a physicochemical way (*e.g.* optical, electrochemical) that transforms the signal resulting from the interaction of the compound with the test substance into another signal (*i.e.*, transducers) that can be more easily measured and quantified. Third, an associated electronic or signal processor which then displays the results of the interaction in a user-friendly way. The present invention provides a method of assessing whether a test compound functions as a ligand for a voltage-dependent ion channel, the method comprising: (i) providing a cell comprising a nucleotide sequence encoding a voltage-dependent ion channel fusion subunit bound to a nucleotide sequence encoding at least one bioluminescent donor molecule and/or bound to a nucleotide sequence encoding at least one acceptor molecule; (ii) contacting said cell with a test compound; and (iii) determining the resultant reaction or interaction and output. The current invention also provides biosensor comprising a nucleotide sequence encoding a voltage-dependent ion channel fusion subunit bound to a nucleotide sequence encoding at least one bioluminescent donor molecule and/or bound to a nucleotide sequence encoding at least one acceptor molecule. Still further the invention provides bioluminescence resonance energy transfer system comprising a nucleotide sequence encoding a voltage-dependent ion channel fusion subunit bound to a nucleotide sequence encoding at least one bioluminescent donor molecule and/or bound to a nucleotide sequence encoding at least one acceptor molecule.

In another embodiment, the methods of screening according to the current invention are used for drug discovery and/or development. Also contemplated within the scope of invention is a method of making a pharmaceutical composition comprising (i) performing the method according to the current invention (ii) identifying a test compound that interacts with voltage-dependent ion channel; and (iii) combining said test compound with a pharmaceutically acceptable carrier.

The fusion subunits of the present invention may be further useful for studying the effect of other stimuli than the action of a modulator (inhibitor or activator) of the channel, such as the effect of temperature and/or radiofrequences on said channel. Actually, the conformational change of the fusion channel subunit(s) that triggers the BRET emission may be obtained by contacting said channel with a modulator thereof, or by submitting said channel to another stimulus, such as an increase in temperature or an irradiation with radiofrequences.

## EXAMPLES

### EXAMPLE 1

### Example 1.1 Methods of analyzing in real time the TRP channel activation on live cells

Voltage-dependent ion channel such as TRPV1 and TRPV3 were used to study the effects of specific agonist and temperature increase on TRP channel activation. Pharmacology of TRPV1 for example, has been well characterized and various specific antagonists thereof are commercially available. On the other hand, TRPV1 has been studied and is known to open at 43°C.

Two methods were used. The first method used was a direct method of assessing the activation of channels such as TRP. This method consists in measuring BRET signals from voltage-dependent ion channels, TRPV1, TRPV3 or TRPV4, which N- and C-termini have been fused to YFP and to luciferase, respectively (Figure 3). The activation of the channels following for example agonist exposure or cell culture temperature increase results in a change of the conformation of the monomer subunits which result in a modification of either or both the distance and the orientation of the donor and acceptor molecules in N- and C-terminal ends of the channels. Another method consist in measuring BRET signals from two voltage-dependent ion channels subunits, each ones being fused to either the YFP or the luciferase at one of each monomer extremity.

### Example 1.2: Construction of expression vectors

Expression vectors were constructed using intramolecular probes YFP-hTRPV1-RLuc and YFP-hTRPV3-RLuc, wherein protein sequences of human channel subunits TRPV1 et TRPV3 (hTRPV1, hTRPV3) are inserted between *Renilla Luciferase,* in 3', and YFP, in 5', according to the strategy presented in Figure 4.

Constructs YFP-hTRPV1-RLuc and YFP-hTRPV3-RLuc have been obtained by amplification of hTRPV1 et hTRPV3 cDNA via PCR, using specific primers allowing fusion of amplicons between sequences of YFP and RLuc in a pcDNA3.1(+) YFP-RLuc vector (Figure 4). cDNA encoding hTRPV1 et hTRPV3 were used as matrices, and were provided from database PlasmID (Harvard Medical School, U.S.A). Sense and antisense primer sequences that were used to amplify by PCR hTRPV1 and hTRPV3 are as follows:

hTRPV1 sense (S) - SEQ ID NO: 1 5'TGTGTACCGGTGAATTCTGGTGGAGGCGGATCTATGAAGAAATGGAGCAGCACAGACT-3'

hTRPV1 anti-sense (AS) - SEQ ID NO: 2 5'CACCAGAATTCACCGGTACCTTCTCCCCGGAAGCGGCAGGACTC-3'

hTRPV3 sense (S) - SEQ ID NO: 3 5'TGTGTACCGGTGAATTCTGGTGGAGGCGGATCTATGAAAGCCCACCCCAAGGAGATGG-3'

hTRPV3 anti-sense - SEQ ID NO: 4 5'CACCAGAATTCACCGGTACCACCGAGGTTTCCGGGAATTCCTCG-3'

hTRPM2_Fus AS - SEQ ID NO : 5 5' CACCAGAATTCACCGGTACGTAGTGAGCCCCGAACTCAGCGGC- 3'

Fusion_hTRPM2_S - SEQ ID NO: 6 5'TGTGTACCGGTGAATTCTGGTGGAGGCGGATCTATGGAGCCCTCAGCCCTGAGGAAAGC-3'

hTRPV4 Fus AS - SEQ ID NO: 7 5'CACCAGAATTCACCGGTACGAGCGGGGCGTCATCAGTCCTCCACTTGCG-3'

Fusion_hTRPV4_S : SEQ ID NO:8 5'TGTGTACCGGTGAATTCTGGTGGAGGCGGATCTATGGCGGATTC-CAGCGAAGGCCCCG- 3'

YFP sense - SEQ ID NO: 9 5'-TGTCTAAGCTTGGATCCGCCACCATGGTGAGCAAGGGCGAGGAGCTGTTCACC-3'

Using the above primers, the amino acid sequence of the region linking YFP to hTRPV1/3 in N-terminal and linking hTRPV1/3 to RLuc in C-terminal of the fusion channel subunit, was VPVNSGGGS (SEQ ID NO: 10).

The PCR reaction was performed in a volume of 50 μl in a buffer comprising 1 μM of each primers, 250 μM dNTP, 5 μl PCR buffer Phusion HF II 10X (Thermoscientific), 5% DMSO, 1 U Phusion enzyme HF II 10X (Thermoscientific), and 25 ng matrix DNA. The PCR was performed in a thermocyclor (Rotor Gene Q, Qiagen) according to program listed in the following Table 13.

Table 13: program of PCR amplication of hTRPV1 and hTRPV3

| Stages | Temp (°C) | Time | Number of cycles |
|---|---|---|---|
| Initial denaturation | 95 | 1 min | 1 |
| Denaturation | 95 | 45 sec | |
| Hybridation | 55 | 45 sec | 25 |
| Elongation | 72 | 2 min | |
| Final elongation | 72 | 4 min | 1 |

PCR products were then purified on agarose gel using the QIAquick Gel Extraction kit (Qiagen). PCR products and vector pcDNA3.1(+) YFP-RLuc were then digested either by the Age I enzyme for the cloning of hTRPV1, or by EcoRI enzyme in the case of hTRPV3 cloning. The vector pcDNA3.1(+) YFP-RLuc was also dephosphorylated using a phosphatase alcaline enzyme (Thermoscientific). DNAs were then prepared and purified on a column using QIAquick PCR purification Kit (Qiagen), and further quantified on gel. Ligation of the amplicons of PCR at the restriction site within the opened vector pcDNA3.1(+) YFP-RLuc was then performed by incubating 50 ng of opened vectors with 20 to 100 ng insert (molar ratio greater or equal to 3:1) in 12 μl of ligation buffer 1X (Thermoscientific) comprising 1,2 mM ATP and 1 U of T4 DNA ligase (Thermoscientific), at 23 °C for 2 hours. The ligation mix was then used to transform *Escherichia coli* DH5α by thermal choc. 100 μl of competent DH5α bacteria were incubated on ice in presence of 12 μl of the ligation mix during 1 hour. Bacteria were then put at 42 °C for 20 sec before being transferred back to ice for 2 minutes. They were then placed under agitation at 200 RPM, at 37 °C for 1 hour after addition of 500 μl *Luria-Bertani* medium (LB) (Fischer Scientific), and spread on LB-Agar box containing 100 μg/ml of ampicillin allowing to select successfully transformed bacteria.

The insertion of the sequence hTRPV1/3 in between YFP and hRLuc was not orientated. It was thus necessary to further screen colonies to select those that contained inserts hTRPV1/3 in the correct orientation. This was performed using the PCR technique using the « sense » primer which was capable of hybridizing in 5' of the coding sequence of YFP and using the antisense primer hybridizing in 5' of the non-coding sequence of hTRPV1/3. This analytical PCR protocol was similar to that of above described PCR except for the Dream Taq polymerase (thermoscientific) which was used during 30 cycles of PCR with an initial denaturation step of 5 min at 95 °C allowing to destroy bacteria and releasing plasmid DNA. Colonies which contained cDNA of hTRPV1/3 in phase with YFP allowed amplication only. A positive clone is thus cultured in 100 ml of LB medium (16 hours, 37 °C). Plasmid DNA is prepared by alcaline lysis of bacteria and purification using the midiprep kit (Qiagen). DNA so obtained was then double checked by sequencing.

The same protocol was successfully used to construct expression vectors respectively comprising the hTRPV4 and hTRPM2 sequences.

### Example 1.3: Cell culture and transfection

Human cell line HEK293T which originated from kidney embryonic cells, had been selected for this experiment. These cells were cultivated in DMEM medium (Dulbecco's Modified Eagle's Medium) containing GLUTAmax (Invitrogen), 10% fetal calf serum, 100 units/ml of penicillin and streptomycin (Invitrogen), 1 mM of sodium pyruvate (Invitrogen). These cells were placed in culture flasks T25 or T75 up to 70-90% confluence on the day of transfection. Transient transfections were performed using polyethylenimin (linear PEI, Polysciences, Inc., Warrington, USA) in Opti-MEM medium (Invitrogen) with a ratio PEI:DNA of 4:1. Transfection of the cells cultured in a T75 flask required 15 μg of total DNA placed in 700 μl of opti-MEM medium (5 minutes, RT) wherein 60 μl of 1 μg/μl PEI are added. The mixture was vortexed 5 sec and incubated at RT during 20 min before being added to the cell culture. The next day, cells were collected using trypsin-EDTA 1% and cultured for 24 hours either in 96 well plaques ($1.10^5$ cells/well) for dose-response experiments, or in 24 well plaques containing glass slides of 12 mm de diameter ($1.10^6$ cells/well) for all other experiments. Both types of support had been treated with L-polylysine (Sigma).

### Example 1.4: Measure of intracellular calcium by Fura 2-AM

HEK293T Cells were transfected with DNA encoding wild-type TRPV1 or YFP-hTRPV1-rLuc. After 24 hours, 150 000 cells are plated in a 96 wells-plates in DMEM medium. Further an additional 24 hours, the medium is replaced by HBSS buffer (140 mM NaCl, 4.2 mM KCl, 0.4 mM $Na_2HPO_4$, 0.5 mM $NaH_2PO_4$, 0.3 mM $MgCl_2$, 0.4 mM $MgSO_4$, 1 mM $CaCl_2$, 20 mM Hepes, 5 mM glucose, pH 7.4) comprising 2 μM Fura 2-AM (Tocris) and 0.02 % of pluronic acid, and cells were incubated at 37 °C for 1 hour without any light stimulation. Reading of the fluorescent signal was performed with multifonction Flexstation III plaque reader (Molecular device) preheated at 37 °C. The light signal has been integrated at 505 nm for 1 second after having sequentially excited the probe at 340 nm and 380 nm. After 100 seconds of reading, 1 μM of capsaïcin was added to the cellular medium. The results presented on figure 7A present the ratio of light intensity as obtained at 505 nm during the excitation at 340 nm and at 380 nm ($I_{340}/I_{380}$).

The same protocol was successfully used to measure intracellular calcium in cells similarly transfected with wild-type TRPV3 or YFP-hTRPV3-rLuc.

### Example 1.5: Measure of BRET signal

Readings of BRET signals in experiments of dose-response were realized in of 96 opaque wells-plates with a multifonction Flexstation III plaque reader (Molecular device) preheated at 37 °C. 36-48 hours after the transfection, the cellular medium was replaced with PBS containing 0.2% BSA and various concentrations of capsaicin ligand (Tocris). After 5 minutes of incubation at 37 °C, the substrate cœlenterazine H (Nanolight Technology) was added to a final concentration of 5 μM and the light signal was integrated for 1 second sequentially at 480 nm and 530 nm, *e.g.*, the wavelengths of emission of luciferase and YFP, respectively. A third reading was performed at 300 nm in order to measure the background signal of the device. The raw BRET signal was calculated by the following equation:

BRET = $(I_{530}-I_{300})/(I_{480}-I_{300})$, wherein I is the light intensity as obtained at the given wavelength.

The BRET signal was then corrected by removing the BRET signal obtained during the light emission of luciferase when the protein TRPV1-rLuc was expressed in absence of YFP.

### Example 1.6: Analysis of the data

The results of BRET obtained with variations of temperatures and with the Fura 2-AM probe have been analyzed on Excel (Microsoft). The results obtained in dose-response experiments have been analyzed with the GraphPad Prism software v6.00 (GraphPad Software Inc, La Jolla, CA, USA).

### Example 2: Direct measuring methods of activation/inhibition of a voltage-dependent cation channel

In order to study the activation/inhibition of the voltage-dependent cation channel fusion subunit, for example of the family TRPV (vanilloid) channel, such as TRPV1, by the temperature or a chemical modulator, expression vectors were first constructed encoding fusion channel subunit wherein sequences of TRPV1 were taken in sandwich between N-terminal YFP fused subunit and C-terminal RLuc fused subunit.

**Example 2.1 Characterization of TRPV1 BRET probe**

In order to study the behaviour of the intramolecular BRET probe, *i.e.*, YFP-hTRPV1-rLuc over temperature, HEK293T cells were transfected using the expression vector encoding the fusion subunits. 24 hours after transfection, cells were progressively heated from 29 to 50 °C with a Peltier thermostat, and the BRET signals were measured using known techniques.

The analysis of the results showed that the BRET ratio did not change between 29 and 34 °C, and slightly increased between 34 °C and 43 °C. Over 43 °C, an important decrease of the BRET value was observed (Figure 5). Temperature of 43 °C corresponded to the threshold of opening of the TRPV1 channel (Clapham, 2003 Nature 426, 517-524 (4 December 2003) | doi:10.1038/nature02196).

The same cells were then pre-incubated with two antagonists of TRPV1, namely 6 iodo-CAPS and 2,2-Diphenyltetrahydrofuran which modify the initial value of net BRET of the probe YFP-hTRPV1-Luc. The ratio of net BRET as initially measured at 29.5 °C was of 0.43 in control conditions, and of 0.28 in presence of 6 iodo-CAPS and of 0.93 in presence 2,2-Diphenyltetrahydrofuran. These important changes of the initial BRET level could have reflected a transition of the conformation state of the TRPV1 channels to a state having a lesser thermosensitivity. According to this hypothesis, a completely different behavior of the probe was observed with variations of temperature in presence of 6 iodo- CAPS or 2,2-Diphenyltetrahydrofuran. In both cases, BRET signal increased between 32 and 37 °C, and plateaued at 52 °C in the case of 2,2-Diphenyltetrahydrofuran, and only decreased as from 48 °C for 6 iodo-CAPS (Figure 5).

The sensitivity of the probe YFP-hTRPV1-rLuc to chemical activators was also assessed. The BRET signal in HEK293T cells expressing the YFP-hTRPV1-rLuc fusion protein was measured after incubation of the cells in presence of different concentrations of capsaïcin. The BRET signal was function of the concentration of capsaïcin, thereby showing a significant increase of the ratio with an EC50 of $650 \pm 33$ nM (Figure 6) showing that the effect of capsaicin on to the probe. In this case, the ratio of initial BRET signal was about 0.28 but could not be compared to the values obtained in the preceding experiments, since it was performed with a different device system. As expected, known hTRPV1 antagonists like AMG517 or Capsazepine induce a right-shift of the Capsaicine dose-response curve, which is in agreement with the notion that these molecules decrease TRPV1 activation threshold by Capsaicin (Figure 6).

The functionality of the fused subunit channels YFP-hTRPV1-rLuc was compared to that of wild-type channels. The flux of calcium was measured in HEK293T cells expressing either the probe BRET YFP-hTRPV1-rLuc, or the wild-type TRPV1, after the addition of 1 μM agonist capsaïcin. We have used a radiometric probe Fura 2-AM which has spectral properties compatible with excitation spectrum of YFP. The addition of capsaïcin resulted in a rapid and important increase of intracellular calcium in cells expressing wild-type TRPV1 as well as in cells expressing YFP-hTRPV1-rLuc with similar kinetic and amplitude (Figure 7A). No calcium increase has been measured in HEK293T cells which are transfected with an empty vector after the addition of capsaicin (Figure 7B), thereby confirming that the signal as measured in Figure 7A is dependent of the ectopic expression of TRPV1.

**Example 2.2 Characterization of the TRPV3 BRET probe**

The sensitivity of the probe YFP-hTRPV3-rLuc to temperature was assessed as described above in example 2.1 for YFP-hTRPV1-rLuc.The sensitivity of the probe YFP-hTRPV3-rLuc to chemical activators was also assessed. The kinetic of the evolution of the BRET signal in HEK293T cells expressing the YFP-hTRPV3-rLuc fusion protein was measured at 30°C. At a defined time, a TRPV3 activator, such as 2-Aminoethyl diphenylborinate, was added in the culture media producing an increase in the BRET value within seconds (Figure 8).

**Example 2.3 Characterization of the TRPV4 BRET probe**

The sensitivity of the probe YFP-hTRPV4-rLuc to temperature was assessed as described above in example 2.1 for YFP-hTRPV1-rLuc.

Activation/inhibition of other voltage-dependent cation channels belonging to TRPC (canonical) channel, TRPV (Vaniloid), TRPM (melastatin) channel, TRPA (ankyrin) channel, TRPP (polycystin) channel, or TRPML (mucolipin) channel is characterized similar to that of TRPV1, TRPV3 or TRPV4 channel.

**Example 3: Direct measuring methods of activation/inhibition of voltage-dependent anion channel**

A similar strategy as that of Example 2 for TRPV1 is used to study activation of inhibition of voltage-dependent ion channel such as sodium gated sodium channel (SCN channel), calcium gated channel (CACNA channel), CATPSER channel, CNG channel, voltage dependent potassium channel (KCN channel), and/or hydrogen voltage-gated ion channel.

**Claims**

1. A nucleic acid comprising a nucleotide sequence encoding a voltage-dependent ion channel fusion subunit bound to a nucleotide sequence encoding at least one bioluminescent donor molecule and bound to a nucleotide sequence encoding at least one fluorescent acceptor molecule, wherein said bioluminescent donor molecule and fluorescent acceptor molecule are selected so that the emission spectrum of the bioluminescent donor molecule overlaps with the absorbance spectrum of the acceptor molecule, such that the light energy delivered by the bioluminescent donor molecule is at a wavelength that is able to excite the fluorescent acceptor molecule, wherein the bioluminescent donor molecule is bound to C-terminal of channel subunit and fluorescent acceptor molecule is bound to N-terminal of said channel subunit, or the bioluminescent donor molecule is bound to N-terminal of channel subunit and fluorescent acceptor molecule is bound to C-terminal of said channel subunit, and wherein said voltage-dependent ion channel subunit is a subunit of a voltage-dependent cation channel or voltage-dependent anion channel and comprises 2 to 24 transmembrane domains.

2. Nucleic acid of claim 1, wherein said voltage-dependent cation channel subunit is a subunit of a transient receptor potential (TRP) channel, and comprises 6 transmembrane domains, 2 intracellular loops, 1 transmembrane loop, and intracellular N- and C-termini.

3. Nucleic acid according to claim 1, wherein the bioluminescent donor molecule and the fluorescent acceptor molecule may be optionally bound to N-terminal and C-terminal of said channel subunit via a linker sequence.

4. Nucleic acid of any one of the preceding claims, wherein the bioluminescent donor molecule is a protein chosen among luciferase, chosen among Renilla luciferase, Firefly luciferase, Coelenterate luciferase, North American glow worm luciferase, click beetle luciferase, a railroad worm luciferase, Gaussia luciferase, Aequorin, Arachnocampa luciferase, or a biologically active variant or fragment of any one, or non luciferase bioluminescent protein chosen among β-galactosidase, lactamase, horseradish peroxydase, alkaline phosphatase, β-glucuronidase, or β-glucosidase, and wherein the acceptor molecule is a protein chosen among green fluorescent protein (GFP), variant of green fluorescent protein (GFP10), blue fluorescent protein (BFP), cyan fluorescent protein (CFP), yellow fluorescent protein (YFP), enhanced GFP (EGFP), enhanced CFP (ECFP), enhanced YFP (EYFP), GFPS65T, mAmetrine, LSS-mOrange, LSS-mKate, Emerald, Topaz, GFPuv, destabilised EGFP (dEGFP), destabilised ECFP (dECFP), destabilised EYFP (dEYFP), HcRed, t-HcRed, DsRed, DsRed2, mRFPl, pocilloporin, Renilla GFP, Monster GFP, paGFP, Kaede protein or a Phycobiliprotein, or a biologically active variant or fragment of any one thereof, or wherein the acceptor molecule is Alexa, fluor dye, Bodipy dye, Cy dye, fluorescein, dansyl, umbelliferone, fluorescent microsphere, luminescent nanocrystal, Marina blue, Cascade blue, Cascade yellow, Pacific blue, Oregon green, Tetramethylrhodamine, Rhodamine, Texas red, rare earth element chelates, or any combination or derivatives thereof.

5. An expression vector comprising a nucleotide sequence encoding the channel fusion subunit as defined in any one of the preceding claims, operably linked to a promoter and optionally to an enhancer, wherein said promoter is CMV promoter, RSV promoter, SV40 promoter, adenovirus promoter, adenovirus E1A, Heat Shock protein promoter, a promoter from *Mycobacteria* genes and RNA, *Mycobacterium bovis* MPB70, MPB59, or MPB64 antigen promoter, PI promoter from bacteriophage Lambda, a tac promoter, a trp promoter, a lac promoter, a lacUV5 promoter, an Ipp promoter, a $P_L\lambda$ promoter, a $P_R\lambda$ promoter, a rac5 promoter, a β-lactamase, a recA promoter, a SP6 promoter, a T7 promoter, a metallothionine promoter, a growth hormone promoter, a hybrid promoter between a eukaryotic promoter and a prokaryotic promoter, ubiquitin promoter, E2F, CEA, MUC1/DF3, α-fetoprotein, erb-B2, surfactant, tyrosinase, PSA, TK, p21, hTERT, hKLK2, probasin or a cyclin gene derived promoter and wherein said enhancer is selected from immediate early enhancer, β-actin, or an adenovirus inverted terminal repeats (ITR), or wherein said expression vector is a DNA or RNA vector, capable of transforming eukaryotic host cells and effecting stable or transient expression of said channel fusion subunit, and wherein said vector is a plasmid, a virus like adenovirus, adeno associated virus (AVV), lentiviral, Epstein-Barr, Herpes Simplex, Papilloma, Polyoma, Retro, SV40, Vaccinia, any retroviral vector, influenza viral vector and other non-viral vectors including naked DNA, or liposomes.

6. A recombinant cell comprising the expression vector of claim 5, wherein said channel fusion subunit is expressed, and is able to co-assemble with other homomeric or heteromeric channel subunits *in vitro* and *in vivo* to form a functional channel.

7. A process for the production of a channel fusion subunit as defined in anyone of claims 1-4 comprising culturing said recombinant cell of claim 6, and expressing the TRP channel fusion subunit of any one of claims 1-5.

8. A fusion subunit encoded by the nucleic acid of any one of claims 1-4, wherein at least one N-terminal extremity or one C-terminal extremity of said subunit is bound to at least one bioluminescent donor protein and at least one acceptor protein.

9. A cell free composition comprising the fusion subunit of claim 8, wherein said fusion protein is embedded in a lipid bilayer, preferably in a bilayer of a liposome.

10. A process for preparing a cell free composition of claim 9, comprising obtaining a recombinant cell of claim 7 and disrupting the membrane of the cells.

11. Method of screening in real time a compound candidate capable of activating or inhibiting a channel, comprising:

- contacting the candidate with the recombinant host cell of claim 6, or a cell free composition of claim 9,
- providing a substrate of the bioluminescent donor molecule; and
- measuring the variation of BRET signal.

12. Method of claim 11, wherein the substrate is firefly luciferin, bacterial luciferin, dinoflagellate luciferin, imidazolopyrazine vargulin, coelenterazine, or analogues/derivatives thereof.

13. Kit for screening agonist or inhibitor compound candidate of voltage-dependent ion channel comprising a nucleic acid of any one of claims 1-4, a vector of claim 5, a recombinant host cell of claim 6, or a cell-free composition of claim 9.

**Patentansprüche**

1. Nukleinsäure, umfassend eine Nukleotidsequenz, die eine spannungsabhängige Ionenkanalfusions-Untereinheit codiert, die an eine Nukleotidsequenz gebunden ist, die mindestens ein biolumineszierendes Spendermolekül codiert, das an eine Nukleotidsequenz gebunden ist, die mindestens ein fluoreszierendes Empfängermolekül codiert, wobei das biolumineszierende Spendermolekül und das fluoreszierende Empfängermolekül derart ausgewählt sind, dass das Emissionsspektrum des biolumineszierenden Spendermoleküls mit dem Absorptionsspektrum des Empfängermoleküls überlappt, so dass die Lichtenergie, die vom biolumineszierenden Spendermolekül geliefert wird, eine Wellenlänge aufweist, die dazu in der Lage ist, das fluoreszierende Empfängermolekül zu erregen, wobei das biolumineszierende Spendermolekül an den C-Terminus der Kanal-Untereinheit gebunden ist und das fluoreszierende Empfängermolekül an den N-Terminus der Kanal-Untereinheit gebunden ist oder das biolumineszierende Spendermolekül an den N-Terminus der Kanal-Untereinheit gebunden ist und das fluoreszierende Empfängermolekül an den C-Terminus der Kanal-Untereinheit gebunden ist, und wobei die spannungsabhängige Ionenkanal-Untereinheit eine Untereinheit eines spannungsabhängigen Kationenkanals oder spannungsabhängigen Anionenkanals ist und 2 bis 24 Transmembrandomänen umfasst.

2. Nukleinsäure nach Anspruch 1, wobei die spannungsabhängige Kationenkanal-Untereinheit eine Untereinheit eines Transient Receptor Potential (TRP)-Kanals ist und 6 Transmembrandomänen, 2 intrazelluläre Schleifen, 1 Transmembranschleife und intrazelluläre N- und C-Termini umfasst.

3. Nukleinsäure nach Anspruch 1, wobei das biolumineszierende Spendermolekül und das fluoreszierende Empfängermolekül optional an den N-Terminus und den C-Terminus der Kanal-Untereinheit über eine Linkersequenz gebunden sein können.

4. Nukleinsäure nach einem der vorhergehenden Ansprüche, wobei das biolumineszierende Spendermolekül ein Protein ist, ausgewählt aus Luciferase, ausgewählt aus Renilla-Luciferase, Firefly-Luciferase, Hohltier-Luciferase, nordamerikanischer Glühwurm-Luciferase, Schnellkäfer-Luciferase, Apfelfliegen-Luciferase, Gaussia-Luciferase, Aequorin, Arachnocampa-Luciferase oder einer biologisch aktiven Variante oder einem Fragment davon, oder biolumineszierendes Nicht-Luciferase-Protein, ausgewählt aus β-Galactosidase, Lactamase, Meerettich-Peroxydase, alkalischer Phosphatase, β-Glucuronidase oder β-Glucosidase, und wobei das Empfängermolekül ein Protein ist, ausgewählt aus grün fluoreszierendem Protein (GFP), einer Variante von grün fluoreszierendem Protein (GFP10), blau fluoreszierendem Protein (BFP), Cyan fluoreszierendem Protein (CFP), gelb fluoreszierendem Protein (YFP), erhöhtem GFP (EGFP), erhöhtem CFP (ECFP), erhöhtem YFP (EYFP), GFPS65T, mAmetrin, LSS-mOrange, LSS-mKate, Smaragd, Topas, GFPuv, destabilisiertem EGFP (dEGFP), destabilisiertem ECFP (dECFP), destabilisiertem EYFP (dEYFP), HcRed, t-HcRed, DsRed, DsRed2, mRFP1, Pocilloporin, Renilla-GFP, Monster-GFP, paGFP, Kaede-Protein oder Phycobiliprotein oder einer biologisch aktiven Variante oder einem Fragment davon, oder wobei das Empfängermolekül Alexa, Fluorfarbstoff, Bodipy-Farbstoff, Cy-Farbstoff, Fluorescein, Dansyl, Umbelliferon, fluoreszierende Mikrosphäre, lumineszierendes Nanokristall, Marineblau, Kaskadenblau, Kaskadengelb, Pazifikblau, Oregongrün, Tetramethylrhodamin, Rhodamin, Texasrot, Seltenerdchelate oder alle Kombinationen oder Derivate davon ist.

**5.** Expressionsvektor, umfassend eine Nukleotidsequenz, die die Kanalfusions-Untereinheit codiert, wie in einem der vorhergehenden Ansprüche definiert, in Betrieb verbunden mit einem Promoter oder optional einem Verstärker, wobei der Promoter ein CMV-Promoter, RSV-Promoter, SV40-Promoter, Adenovirus-Promoter, Adenovirus E1A, Hitzeschockprotein-Promoter, ein Promoter von *Mycobacteria-Genen* und RNA, *Mycobacterium bovis* MPB70-, MPB59- oder MPB64-Antigenpromoter, P1-Promoter der Bakteriophage Lambda, ein tac-Promoter, ein trp-Promoter, ein lac-Promoter, ein lacUV5-Promoter, ein Ipp-Promoter, ein $P_L\lambda$,-Promoter, ein $P_R\lambda$-Promoter, ein rac5-Promoter, eine β-Lactamase, ein recA-Promoter, ein SP6-Promoter, ein T7-Promoter, ein Metallothionin-Promoter, ein Wachstumshormon-Promoter, ein Hybrid-Promoter zwischen einem eukaryotischen Promoter und einem prokaryotischen Promoter, Ubiquitin-Promoter, E2F, CEA, MUC1/DF3, α-Fetoprotein, erb-B2, Tensid, Tyrosinase, PSA, TK, p21, hTERT, hKLK2, Probasin oder ein Cyclingen-abgeleiteter Promoter ist, und wobei der Verstärker ausgewählt ist aus einem unmittelbar frühen Verstärker, β-Actin, oder einem Adenovirus-Inverted Terminal Repeats (ITR), oder wobei der Expressionsvektor ein DNA- oder RNA-Vektor ist, der dazu in der Lage ist eukaryotische Wirtszellen umzuwandeln und eine stabile oder vorübergehende Expression der Kanalfusions-Untereinheit zu bewirken, und wobei der Vektor ein Plasmid, ein virusähnliches Adenovirus, ein adenoassoziiertes Virus (AVV), lentiviral, Epstein-Barr, Herpes Simplex, Papilloma, Polyoma, Retro, SV40, Vaccinia, jeder retrovirale Vektor, Grippevirus-Vektor und andere nicht virale Vektoren, darin eingeschlossen nackte DNA oder Liposome ist.

**6.** Rekombinante Zelle, umfassend den Expressionsvektor von Anspruch 5, wobei die Kanalfusions-Untereinheit exprimiert ist und dazu in der Lage ist, sich mit anderen homomeren oder heteromeren Kanal-Untereinheiten *in vitro* und *in vivo* zusammenzufügen, um einen funktionellen Kanal zu bilden.

**7.** Verfahren zur Herstellung einer Kanalfusions-Untereinheit, wie nach einem der Ansprüche 1 bis 4 definiert, umfassend Kultivieren der rekombinanten Zelle nach Anspruch 6 und Exprimieren der TRP-Kanalfusions-Untereinheit nach einem der Ansprüche 1 bis 5.

**8.** Fusions-Untereinheit, codiert durch die Nukleinsäure nach einem der Ansprüche 1 bis 4, wobei mindestens ein N-terminales Ende oder ein C-terminales Ende der Untereinheit an mindestens ein biolumineszierendes Spenderprotein und mindestens ein Empfängerprotein gebunden ist.

**9.** Zellfreie Zusammensetzung, umfassend eine Fusions-Untereinheit nach Anspruch 8, wobei das Fusionsprotein in eine Lipid-Doppelschicht, vorzugsweise in eine Doppelschicht eines Liposoms eingebettet ist.

**10.** Prozess zur Herstellung einer zellfreien Zusammensetzung nach Anspruch 9, umfassend Erhalten einer rekombinanten Zelle von Anspruch 7 und Durchbrechen der Membran der Zellen.

**11.** Verfahren zum Echtzeit-Screenen eines Verbindungskandidaten, der dazu in der Lage ist, einen Kanal zu aktivieren oder zu hemmen, umfassend:

- In-Kontakt-Bringen des Kandidaten mit der rekombinanten Wirtszelle nach Anspruch 6 oder einer zellfreien Zusammensetzung nach Anspruch 9,
- Bereitstellen eines Substrats der biolumineszierenden Spendermolekül; und
- Messen der Variation des BRET-Signals.

**12.** Verfahren nach Anspruch 11, wobei das Substrat Firefly-Luciferin, bakterielle Luciferin, Dinoflagellat-Luciferin, Imidazolopyrazine-Vargulin, Coelenterazin oder Analoga/Derivate davon ist.

**13.** Kit zum Screenen eines Agonisten- oder Inhibitorverbindungs-Kandidaten eines spannungsabhängigen Ionenkanals, umfassend eine Nukleinsäure nach einem der Ansprüche 1 bis 4, einen Vektor nach Anspruch 5, eine rekombinante Wirtszelle nach Anspruch 6 oder eine zellfreie Zusammensetzung nach Anspruch 9.

**Revendications**

**1.** Acide nucléique comprenant une séquence nucléotidique codant pour une sous-unité de fusion de canal ionique dépendant de la tension liée à une séquence nucléotidique codant pour au moins une molécule donneuse bioluminescente et liée à une séquence nucléotidique codant pour au moins une molécule acceptrice fluorescente, dans lequel lesdites molécule donneuse bioluminescente et molécule acceptrice fluorescente sont sélectionnées de telle sorte que le spectre d'émission de la molécule donneuse bioluminescente chevauche le spectre d'absorbance de la molécule acceptrice, de façon que l'énergie lumineuse délivrée par la molécule donneuse bioluminescente soit à une longueur d'onde qui est capable d'exciter la molécule acceptrice fluorescente, dans lequel la molécule donneuse bioluminescente est liée à l'extrémité C-terminale de la sous-unité de canal et la molécule acceptrice fluorescente est liée à l'extrémité N-terminale de ladite sous-unité de canal, ou la molécule donneuse bioluminescente est liée à l'extrémité N-terminale de la sous-unité de canal et la molécule acceptrice fluorescente est liée à l'extrémité C-terminale de ladite sous-unité de canal, et dans lequel ladite sous-unité de canal ionique dépendant de la tension est une sous-unité d'un canal cationique dépendant de la tension ou d'un canal anionique dépendant de la tension et comprend 2 à 24 domaines transmembranaires.

2. Acide nucléique selon la revendication 1, dans lequel ladite sous-unité de canal cationique dépendant de la tension est une sous-unité d'un canal à potentiel de récepteur transitoire (TRP), et comprend 6 domaines transmembranaires, 2 boucles intracellulaires, 1 boucle transmembranaire, et des extrémités N- et C-terminales intracellulaires.

3. Acide nucléique selon la revendication 1, dans lequel la molécule donneuse bioluminescente et la molécule acceptrice fluorescente peuvent être facultativement liées à l'extrémité N-terminale et à l'extrémité C-terminale de ladite sous-unité de canal via une séquence de lieur.

4. Acide nucléique selon l'une quelconque des revendications précédentes, dans lequel la molécule donneuse bioluminescente est une protéine choisie parmi une luciférase, choisie parmi la luciférase de Renilla, la luciférase de luciole, la luciférase cœlentérés, la luciférase du ver luisant nord-américain, la luciférase de taupin, une luciférase de ver de chemin de fer, la luciférase de gaussia, l'équorine, la luciférase d'Arachnocampa, ou un variant ou fragment biologiquement actif de l'une quelconque, ou une protéine bioluminescente non-luciférase choisie parmi la β-galactosidase, la lactamase, la peroxydase de raifort, la phosphatase alcaline, la β-glucuronidase, ou la β-glucosidase, et dans lequel la molécule acceptrice est une protéine choisie parmi la protéine fluorescente verte (GFP), un variant de la protéine fluorescente verte (GFP10), la protéine fluorescente bleue (BFP), la protéine fluorescente cyan (CFP), la protéine fluorescente jaune (YFP), la GFP intensifiée (EGFP), la CFP intensifiée (ECFP), la YFP intensifiée (EYFP), GFPS65T, mAmétrine, LSS-mOrange, LSS-mKate, Emerald, Topaz, GFPuv, l'EGFP déstabilisée (dEGFP), l'ECFP déstabilisée (dECFP), l'EYFP déstabilisée (dEYFP), HcRed, t-HcRed, DsRed, DsRed2, mRFPl, la pocilloporine, la GFP de Renilla, la GFP Monster, paGFP, la protéine Kaede ou une phycobiliprotéine, ou un variant ou fragment biologiquement actifs de l'un quelconque, ou dans lequel la molécule acceptrice est Alexa, un colorant fluoré, un colorant Bodipy, un colorant Cy, la fluorescéine, le dansyle, l'umbelliférone, une microsphère fluorescente, un nanocristal luminescent, le bleu Marina, le bleu Cascade, le jaune Cascade, le bleu Pacific, le vert Oregon, la tétraméthylrhodamine, la rhodamine, le rouge Texas, les chélates d'élément de terre rare, ou n'importe quelle combinaison ou n'importe quel dérivé de ceux-ci.

5. Vecteur d'expression comprenant une séquence nucléotidique codant pour la sous-unité de fusion de canal telle que définie dans l'une quelconque des revendications précédentes, liée de manière fonctionnelle à un promoteur et facultativement à un amplificateur, dans lequel ledit promoteur est un promoteur de CMV, un promoteur de RSV, un promoteur de SV40, un promoteur d'adénovirus, E1A d'adénovirus, un promoteur de protéine de choc thermique, un promoteur provenant de gènes et d'ARN de mycobactéries, un promoteur d'antigène MPB70, MPB59 ou MPB64 de *Mycobacterium bovis,* le promoteur P1 du bactériophage Lambda, un promoteur tac, un promoteur trp, un promoteur lac, un promoteur lacUV5, un promoteur Ipp, un promoteur $P_L\lambda$, un promoteur $P_R\lambda$, un promoteur rac5, une β-lactamase, un promoteur recA, un promoteur SP6, un promoteur de T7, un promoteur de métallothionine, un promoteur d'hormone de croissance, un promoteur hybride entre un promoteur eucaryote et un promoteur procaryote, un promoteur d'ubiquitine, ou un promoteur dérivé d'E2F, de CEA, de MUC1/DF3, de l'α-fétoprotéine, d'erb-B2, d'un surfactant, de tyrosinase, de PSA, de TK, de p21, de hTERT, de hKLK2, de la probasine ou d'un gène de cycline et dans lequel ledit activateur est sélectionné parmi un activateur précoce immédiat, la β-actine ou des répétitions inversées terminales (ITR) d'adénovirus, ou dans lequel ledit vecteur d'expression est un vecteur à ADN ou à ARN, capable de transformer des cellules hôtes eucaryotes et d'effectuer une expression stable ou transitoire de ladite sous-unité de fusion de canal, et dans lequel ledit vecteur est un plasmide, un virus de type adénovirus, un virus adéno-associé (AVV), lentiviral, d'Epstein-Barr, Herpès Simplex, de papillome, de polyome, rétro, SV40, de la vaccine, n'importe quel vecteur rétroviral, un vecteur viral de la grippe et d'autres vecteurs non viraux incluant un ADN nu, ou des liposomes.

6. Cellule recombinante comprenant le vecteur d'expression selon la revendication 5, dans laquelle ladite sous-unité de fusion de canal est exprimée, et est capable de se co-assembler avec d'autres sous-unités de canal homomériques

**EP 3 056 902 B1**

ou hétéromériques *in vitro* et *in vivo* pour former un canal fonctionnel.

7. Procédé de production d'une sous-unité de fusion de canal telle que définie dans l'une quelconque des revendications 1 à 4 comprenant la mise en culture de ladite cellule recombinante selon la revendication 6, et l'expression de la sous-unité de fusion de canal TRP selon l'une quelconque des revendications 1 à 5.

8. Sous-unité de fusion codée par l'acide nucléique selon l'une quelconque des revendications 1 à 4, dans laquelle au moins une extrémité N-terminale ou une extrémité C-terminale de ladite sous-unité est liée à au moins une protéine donneuse bioluminescente ou à au moins une protéine acceptrice.

9. Composition acellulaire comprenant la sous-unité de fusion de la revendication 8, dans laquelle ladite protéine de fusion est incrustée dans une bicouche lipidique, de préférence dans une bicouche d'un liposome.

10. Procédé de préparation d'une composition acellulaire selon la revendication 9, comprenant l'obtention d'une cellule recombinante selon la revendication 7 et la rupture de la membrane des cellules.

11. Procédé de criblage en temps réel d'un composé candidat capable d'activer ou d'inhiber un canal, comprenant :

- la mise en contact du candidat avec la cellule hôte recombinante selon la revendication 6, ou une composition acellulaire selon la revendication 9,
- la fourniture d'un substrat de la molécule donneuse bioluminescente ; et
- la mesure de la variation du signal BRET.

12. Procédé selon la revendication 11, dans lequel le substrat est la luciférine de luciole, une luciférine bactérienne, une luciférine de dinoflagellés, une varguline à base d'imidazolopyrazine, la cœlentérazine, ou les analogues/dérivés de celles-ci.

13. Kit pour le criblage d'un composé candidat agoniste ou inhibiteur de canal ionique dépendant de la tension comprenant un acide nucléique selon l'une quelconque des revendications 1 à 4, un vecteur selon la revendication 5, une cellule hôte recombinante selon la revendication 6, ou une composition acellulaire selon la revendication 9.

**Figure 1A**

**Figure 1B**

**Figure 1C**

$$E = \frac{1}{1 + (r/R_0)^6}$$

FIGURE 2

# FIGURE 3

FIGURE 4A

FIGURE 4B

FIGURE 5

FIGURE 6

FIGURE 7

**FIGURE 8**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 00024878 A
- WO 99049019 A
- US 5229285 A
- US 5219737 A
- US 5843746 A
- US 5196524 A
- US 5670356 A
- WO 2007019634 A
- WO 0146691 A
- US 6949377 A

**Non-patent literature cited in the description**

- **DE LA ROSA et al.** *Journal of Biological Chemistry,* 11 October 2013, vol. 288 (41), 29506-29517
- **J. PERBAL.** A Practical Guide to Molecular Cloning. John Wiley and Sons, 1984
- **J. SAMBROOK et al.** Molecular Cloning: A Laboratory Manual. Cold Spring Harbour Laboratory Press, 1989
- Essential Molecular Biology: A Practical Approach. IRL Press, 1991, vol. 1, 2
- DNA Cloning: A Practical Approach. IRL Press, 1995, vol. 1-4
- Current Protocols in Molecular Biology. Greene Pub. Associates, 1988
- Antibodies: A Laboratory Manual. Cold Spring Harbour Laboratory, 1988
- Current Protocols in Immunology. John Wiley & Sons
- **JOHN E. CALIGAN ; BEN M. DUNN ; HIDDE L. PLOEGH ; DAVID W. SPEICHER ; PAUL T. WINGFIELD.** Current Protocols in Protein Science. Wiley and Sons
- **CLAPHAM.** *Nature,* 04 December 2003, vol. 426, 517-524

SEQUENCE LISTING

<110> UNIVERSITE DE BORDEAUX
INSTITUT POLYTECHNIQUE DE BORDEAUX
CENTRE NATIONAL DE LA RECHERCHE SCIENTIFIQUE

<120> NOVEL VOLTAGE-DEPENDENT ION CHANNEL FUSIONS AND METHOD OF USE THEREOF

<130> 1505-EPO

<140> Not Yet Known
<141> 2015-02-16

<160> 10

<170> PatentIn version 3.3

<210> 1
<211> 58
<212> DNA
<213> human

<400> 1
tgtgtaccgg tgaattctgg tggaggcgga tctatgaaga aatggagcag cacagact          58

<210> 2
<211> 44
<212> DNA
<213> human

<400> 2
caccagaatt caccggtacc ttctccccgg aagcggcagg actc          44

<210> 3
<211> 58
<212> DNA
<213> human

<400> 3
tgtgtaccgg tgaattctgg tggaggcgga tctatgaaag cccaccccaa ggagatgg          58

<210> 4
<211> 44
<212> DNA
<213> human

<400> 4
caccagaatt caccggtacc accgaggttt ccgggaattc ctcg          44

<210> 5
<211> 43
<212> DNA

<213> human

<400> 5
caccagaatt caccggtacg tagtgagccc cgaactcagc ggc          43

<210> 6
<211> 59
<212> DNA
<213> human

<400> 6
tgtgtaccgg tgaattctgg tggaggcgga tctatggagc cctcagccct gaggaaagc          59

<210> 7
<211> 49
<212> DNA
<213> human

<400> 7
caccagaatt caccggtacg agcggggcgt catcagtcct ccacttgcg          49

<210> 8
<211> 59
<212> DNA
<213> human

<400> 8
tgtgtaccgg tgaattctgg tggaggcgga tctatggcgg attccagcga aggcccccg          59

<210> 9
<211> 53
<212> DNA
<213> artificial

<220>
<223> primer sequence

<400> 9
tgtctaagct tggatccgcc accatggtga gcaagggcga ggagctgttc acc          53

<210> 10
<211> 9
<212> PRT
<213> artificial

<220>
<223> primer sequence

<400> 10

Val Pro Val Asn Ser Gly Gly Gly Ser
1               5